(19)

Europäisches Patentamt
European Patent Office
Office européen des brevets

(11)　EP 4 260 908 A1

(12)　**EUROPEAN PATENT APPLICATION**
published in accordance with Art. 153(4) EPC

(43) Date of publication:
**18.10.2023　Bulletin 2023/42**

(21) Application number: **21900693.9**

(22) Date of filing: **03.12.2021**

(51) International Patent Classification (IPC):
*A61P 25/28* (2006.01)　　*C12N 1/20* (2006.01)
*C12Q 1/02* (2006.01)　　*C12Q 1/04* (2006.01)
*A61K 35/74* (2015.01)　　*A61K 35/741* (2015.01)
*A23L 33/135* (2016.01)

(52) Cooperative Patent Classification (CPC):
**A23L 33/135; A61K 35/74; A61K 35/741;
A61P 25/28; C12N 1/20; C12Q 1/02; C12Q 1/04;
Y02A 50/30**

(86) International application number:
**PCT/JP2021/044393**

(87) International publication number:
**WO 2022/118944 (09.06.2022 Gazette 2022/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:　**03.12.2020　JP 2020201008**

(71) Applicants:
• **Otsuka Pharmaceutical Co., Ltd.
Tokyo 101-8535 (JP)**
• **Tokyo Institute of Technology
Tokyo 152-8550 (JP)**

(72) Inventors:
• **SHINKAI Shoji
Sakado-shi, Saitama 350-0288 (JP)**
• **KITAMURA Akihiko
Tokyo 173-0015 (JP)**

• **YAMADA Takuji
Tokyo 152-8550 (JP)**
• **USHIDA Kazunari
Kasugai-shi, Aichi 487-8501 (JP)**
• **TSUCHIDA Sayaka
Kasugai-shi, Aichi 487-8501 (JP)**
• **UEDA Atsushi
Osaka-shi, Osaka 540-0021 (JP)**
• **KOUDA Noriyuki
Osaka-shi, Osaka 540-0021 (JP)**
• **KARIYA Takahiro
Osaka-shi, Osaka 540-0021 (JP)**
• **KAWAHARA Tomohiro
Osaka-shi, Osaka 540-0021 (JP)**
• **KOBAYASHI Yodai
Osaka-shi, Osaka 540-0021 (JP)**

(74) Representative: **Hoffmann Eitle
Patent- und Rechtsanwälte PartmbB
Arabellastraße 30
81925 München (DE)**

(54)　**COMPOSITION FOR PREVENTING OR TREATING DETERIORATION IN BRAIN FUNCTION OR MAINTAINING OR IMPROVING BRAIN FUNCTION**

(57)　　The present invention aims to provide a method for evaluating dementia and a composition for preventing or treating deterioration in brain function, or for maintaining or improving brain function, and compared the gut microbiota of healthy individuals, individuals with mild cognitive impairment, and individuals with Alzheimer's disease. As a result, gut microorganisms were selected, such as microorganisms belonging to the genus Faecalibacterium, that are associated with cognitive function. Furthermore, it was revealed that Faecalibacterium prausnitzii, possessing specific DNA, exhibited an improvement effect against brain function deterioration, such as learning and memory disorders.

## Description

[Technical Field]

The present invention relates to microorganisms for preventing or treating the deterioration in brain function, or for maintaining or improving brain function. Furthermore, the present invention relates to a composition that contains the microorganisms, components of these microorganisms, or a culture of these microbes, as well as to an evaluation method for mild cognitive impairment or Alzheimer's disease.

[Background Art]

The brain, along with the spinal cord, forms the central nervous system, playing a central role in activities mediated by nerves, such as cognition, motion, and life maintenance. Therefore, any deterioration in its function can cause significant disruptions in daily and social life. Particularly, with the rapid aging of society in recent years, the increase in diseases such as Alzheimer's disease (AD), and even its precursor, mild cognitive impairment (MCI), has become a global issue, so that there is a pressing demand for the development of effective methods for their prevention and treatment.

In the current situation, the bidirectional communication between the brain and the gut (gut-brain axis) has been gaining attention in recent years, and it is becoming evident that the microorganisms living in the gut (gut microorganisms, gut microbiota) not only affect the health of their host's body, but also influence brain functions.

For instance, it has been reported that in AD model mice, fluctuations in the gut microbiota due to antibiotic administration influence both brain inflammation associated with the disease and the amount of amyloid-$\beta$ (NPL 1), and that in germ-free AD model mice, which lack gut microbiota, have a reduced amount of brain amyloid-$\beta$ involved in the disease (NPL 2). Moreover, a comparison of the gut microbiota between dementia patients and healthy individuals has revealed that the amount of Bacteroides is reduced in the former (NPL 3).

Moreover, it has been clarified that Faecalibacterium prausnitzii shows preventive and therapeutic effects against stress-induced depressive-like and anxiety-like behaviors in rats (NPL 4). However, there have been no reports regarding the prevention, treatment, and alleviation of symptoms of cognitive decline, as well as the maintenance and improvement of cognitive function.

[Citation List]

[Non Patent Literature]

[NPL 1] Minter MR. et al., Sci Rep., July 21, 2016, 6:30028.
[NPL 2] Harach T. et al, Sci Rep. February 8, 2017, 7:41802.
[NPL 3] Saji N. et al., Sci Rep., January 30, 2019, 9(1): 1008 .
[NPL 4] Hao Z. et al., Psychoneuroendocrinology, June 2019, Volume 104, pp. 132-142

[Summary of Invention]

[Technical Problem]

The present invention aims to find a gut microorganism that is effective against the deterioration in brain function and provide a composition for preventing or treating deterioration in brain function, or for maintaining or improving brain function, that uses the microorganism as an active ingredient. Furthermore, the present invention aims to provide an evaluation method for dementia by selecting gut microorganisms associated with brain functions such as cognitive functions and using the amount of these microorganisms as an indicator.

[Solution to Problem]

The present inventors, in order to achieve the aforementioned object, first compared the gut microbiota between individuals with mild cognitive impairment (MCI) or Alzheimer's disease (AD) patients, and healthy individuals. As a result, gut microorganisms were identified, such as microorganisms belonging to the genus Faecalibacterium, that are associated with the host's cognitive function.

More specifically, it was found that in the gut microbiota, the relative abundance of microorganisms belonging to the genus Faecalibacterium, microorganisms with an unidentified genus in the family Ruminococcaceae, microorganisms belonging to the genus Anaerostipes, microorganisms belonging to the genus CAG-41, microorganisms belonging to the genus Barnesiella, and microorganisms belonging to the genus Ruminococcus, is lower in MCI than in healthy individuals, while the relative abundance of microorganisms belonging to the genus Prevotella is higher in MCI compared to healthy individuals.

In addition, it was found that in the gut microbiota, the relative abundance of microorganisms belonging to the genus Blautia, the genus Fusicatenibacter, the genus Ruminococcus, and the genus Eubacterium is lower in AD patients than in healthy individuals, while the relative abundance of microorganisms belonging to the genus Parabacteroides was higher in AD patients than in healthy individuals.

Moreover, the present inventors successfully isolated and cultured the strain of microorganism belonging to the genus Faecalibacterium (Faecalibacterium prausnitzii), with one strain from a fecal sample of MCM individuals and eleven strains from healthy individuals. Then, these strains were each administered to an AD model mouse, and the degree of improvement in cognitive function decline (learning and memory disorders) in these mice was evaluated using the Y-maze test and passive avoidance test. As a result, improvement effects were observed in two strains of microorganisms specified as Fp14 (accession number NITE BP-03169) and Fp360 (NITE BP-03196).

Furthermore, as a result of comparing the whole genome sequences of the aforementioned two strains and the other ten Fp strains, it was possible to select 28 genes (orthologs) unique to the former, corresponding to the DNA sequences set forth in SEQ ID NOs: 1 to 57 and 422.

In addition, as a result of comparing the whole genome sequences of Fp14 and the other 11 strains, it was possible to select 150 orthologs unique to the former, correspond to the DNA sequences set forth in SEQ ID NOs: 58 to 207.

Moreover, as a result of comparing the whole genome sequences of Fp360 and the other 11 strains, it was possible to select 214 orthologs unique to the former, correspond to the DNA sequences set forth in SEQ ID NOs: 208 to 421.

As above, the present inventors have found a DNA sequence (ortholog) unique to the Fp strain that is capable of exerting an improvement effect on the deterioration of brain function, and have completed the present invention.

That is, the present invention relates to an Fp strain useful for preventing or treating the deterioration in brain function, or for maintaining or improving brain function, a composition containing components of the microorganism, a culture of the microorganism, or any of these as an active ingredient, as well as a method of evaluating dementia using the amount of gut microorganisms associated with the host's cognitive function as an indicator. More specifically, the present invention provides the following.

<1> A composition for preventing or treating deterioration in brain function, or for maintaining or improving brain function, including Faecalibacterium prausnitzii, a component of the microorganism, or a culture of the microorganism, having at least one of the following DNAs (1) to (28), as an active ingredient

(1) a DNA including a nucleotide sequence encoding an amino acid sequence having 40% or more homology to an amino acid sequence encoded by a nucleotide sequence set forth in SEQ ID NO: 22 or 46,
(2) a DNA including a nucleotide sequence encoding an amino acid sequence having 40% or more homology to an amino acid sequence encoded by a nucleotide sequence set forth in SEQ ID NO: 21 or 54,
(3) a DNA including a nucleotide sequence encoding an amino acid sequence having 40% or more homology to an amino acid sequence encoded by a nucleotide sequence set forth in SEQ ID NO: 18 or 55,
(4) a DNA including a nucleotide sequence encoding an amino acid sequence having 40% or more homology to an amino acid sequence encoded by a nucleotide sequence set forth in SEQ ID NO: 9 or 30,
(5) a DNA including a nucleotide sequence encoding an amino acid sequence having 40% or more homology to an amino acid sequence encoded by a nucleotide sequence set forth in SEQ ID NO: 19 or 33,
(6) a DNA including a nucleotide sequence encoding an amino acid sequence having 40% or more homology to an amino acid sequence encoded by a nucleotide sequence set forth in SEQ ID NO: 17 or 34,
(7) a DNA including a nucleotide sequence encoding an amino acid sequence having 40% or more homology to an amino acid sequence encoded by a nucleotide sequence set forth in SEQ ID NO: 3 or 43,
(8) a DNA including a nucleotide sequence encoding an amino acid sequence having 40% or more homology to an amino acid sequence encoded by a nucleotide sequence set forth in SEQ ID NO: 6 or 53,
(9) a DNA including a nucleotide sequence encoding an amino acid sequence having 40% or more homology to an amino acid sequence encoded by a nucleotide sequence set forth in SEQ ID NO: 7 or 35,
(10) a DNA including a nucleotide sequence encoding an amino acid sequence having 40% or more homology to an amino acid sequence encoded by a nucleotide sequence set forth in SEQ ID NO: 2 or 39,
(11) a DNA including a nucleotide sequence encoding an amino acid sequence having 40% or more homology to an amino acid sequence encoded by a nucleotide sequence set forth in SEQ ID NO: 25 or 41,
(12) a DNA including a nucleotide sequence encoding an amino acid sequence having 40% or more homology to an amino acid sequence encoded by a nucleotide sequence set forth in SEQ ID NO: 23 or 50,

(13) a DNA including a nucleotide sequence encoding an amino acid sequence having 40% or more homology to an amino acid sequence encoded by a nucleotide sequence set forth in SEQ ID NO: 24 or 45,

(14) a DNA including a nucleotide sequence encoding an amino acid sequence having 40% or more homology to an amino acid sequence encoded by a nucleotide sequence set forth in SEQ ID NO: 26 or 48,

(15) a DNA including a nucleotide sequence encoding an amino acid sequence having 40% or more homology to an amino acid sequence encoded by a nucleotide sequence set forth in SEQ ID NO: 8 or 40,

(16) a DNA including a nucleotide sequence encoding an amino acid sequence having 40% or more homology to an amino acid sequence encoded by a nucleotide sequence set forth in SEQ ID NO: 4 or 37,

(17) a DNA including a nucleotide sequence encoding an amino acid sequence having 40% or more homology to an amino acid sequence encoded by a nucleotide sequence set forth in SEQ ID NO: 13 or 38,

(18) a DNA including a nucleotide sequence encoding an amino acid sequence having 40% or more homology to an amino acid sequence encoded by a nucleotide sequence set forth in SEQ ID NO: 16 or 56,

(19) a DNA including a nucleotide sequence encoding an amino acid sequence having 40% or more homology to an amino acid sequence encoded by a nucleotide sequence set forth in SEQ ID NO: 14 or 57,

(20) a DNA including a nucleotide sequence encoding an amino acid sequence having 40% or more homology to an amino acid sequence encoded by a nucleotide sequence set forth in SEQ ID NO: 27 or 36,

(21) a DNA including a nucleotide sequence encoding an amino acid sequence having 40% or more homology to an amino acid sequence encoded by a nucleotide sequence set forth in SEQ ID NO: 12 or 42,

(22) a DNA including a nucleotide sequence encoding an amino acid sequence having 40% or more homology to an amino acid sequence encoded by a nucleotide sequence set forth in SEQ ID NO: 15, 31, or 49,

(23) a DNA including a nucleotide sequence encoding an amino acid sequence having 40% or more homology to an amino acid sequence encoded by a nucleotide sequence set forth in SEQ ID NO: 5, 52, or 51,

(24) a DNA including a nucleotide sequence encoding an amino acid sequence having 40% or more homology to an amino acid sequence encoded by a nucleotide sequence set forth in SEQ ID NO: 20 or 44,

(25) a DNA including a nucleotide sequence encoding an amino acid sequence having 40% or more homology to an amino acid sequence encoded by a nucleotide sequence set forth in SEQ ID NO: 1 or 32,

(26) a DNA including a nucleotide sequence encoding an amino acid sequence having 40% or more homology to an amino acid sequence encoded by a nucleotide sequence set forth in SEQ ID NO: 11 or 29,

(27) a DNA including a nucleotide sequence encoding an amino acid sequence having 40% or more homology to an amino acid sequence encoded by a nucleotide sequence set forth in SEQ ID NO: 10 or 47, and

(28) a DNA including a nucleotide sequence encoding an amino acid sequence having 40% or more homology to an amino acid sequence encoded by a nucleotide sequence set forth in SEQ ID NO: 28 or 422.

<2> The composition according to <1>, wherein the Faecalibacterium prausnitzii is Faecalibacterium prausnitzii having the 28 DNAs (1) to (28).

<3> A composition for preventing or treating deterioration in brain function, or for maintaining or improving brain function, including Faecalibacterium prausnitzii, a component of the microorganism, or a culture of the microorganism, having at least one of the following DNAs (1) to (3), as an active ingredient:

(1) a DNA including a nucleotide sequence encoding an amino acid sequence having 40% or more homology to an amino acid sequence encoded by a nucleotide sequence set forth in SEQ ID NO: 22 or 46,

(2) a DNA including a nucleotide sequence encoding an amino acid sequence having 40% or more homology to an amino acid sequence encoded by a nucleotide sequence set forth in SEQ ID NO: 21 or 54,

(3) a DNA including a nucleotide sequence encoding an amino acid sequence having 40% or more homology to an amino acid sequence encoded by a nucleotide sequence set forth in SEQ ID NO: 18 or 55.

<4> The composition according to <3>, wherein the Faecalibacterium prausnitzii is Faecalibacterium prausnitzii having the 3 DNAs (1) to (3).

<5> A composition for preventing or treating deterioration in brain function, or for maintaining or improving brain function, including Faecalibacterium prausnitzii, a component of the microorganism, or a culture of the microorganism, having at least one DNA selected from the following DNA group, as an active ingredient:

A group of 150 DNAs each containing a nucleotide sequence according to SEQ ID NOs: 58 to 207 (Note that the nucleotide sequence according to SEQ ID NO: N is a nucleotide sequence encoding an amino acid sequence having 40% or more homology to the amino acid sequence encoded by the nucleotide sequence set forth in SEQ ID NO: N. N is any of 58 to 207.).

<6> A composition for preventing or treating deterioration in brain function, or for maintaining or improving brain function, including Faecalibacterium prausnitzii, a component of the microorganism, or a culture of the microorganism, having at least one DNA selected from the following DNA group, as an active ingredient:
A group of 214 DNAs each containing a nucleotide sequence according to SEQ ID NOs: 208 to 421 (Note that the nucleotide sequence according to SEQ ID NO: N is a nucleotide sequence encoding an amino acid sequence having 40% or more homology to the amino acid sequence encoded by the nucleotide sequence set forth in SEQ ID NO: N. N is any of 208 to 421.).

<7> The composition according to any one of <1> to <6>, wherein the brain function is cognitive function.

<8> The composition according to any one of <1> to <6>, which is a food composition.

<9> The composition according to any one of <1> to <6>, which is a pharmaceutical composition.

<10> A microorganism which is specified by accession number NITE BP-03169.

<11> A microorganism which is specified by accession number NITE BP-03196.

The present invention also relates to the use of the Faecalibacterium prausnitzii, the microorganism specified by accession number NITE BP-03169, the microorganism specified by accession number NITE BP-03196, a component of these microorganisms, or a culture of these microorganisms, for producing the composition.

The present invention also relates to the use of the Faecalibacterium prausnitzii, the microorganism specified by accession number NITE BP-03169, the microorganism specified by accession number NITE BP-03196, a component of these microorganisms, or a culture of these microorganisms, for preventing or treating deterioration in brain function, or for maintaining or improving brain function.

The present invention also relates to the Faecalibacterium prausnitzii, the microorganism specified by accession number NITE BP-03169, the microorganism specified by accession number NITE BP-03196, a component of these microorganisms, or a culture of these microorganisms, used in preventing or treating deterioration in brain function, or for maintaining or improving brain function.

The present invention also relates to a method for preventing or treating deterioration in brain function, or for maintaining or improving brain function, including administering to a subject an effective amount of the Faecalibacterium prausnitzii, the microorganism specified by accession number NITE BP-03169, the microorganism specified by accession number NITE BP-03196, a component of these microorganisms, or a culture of these microorganisms.

Furthermore, the present invention relates to a method for evaluating dementia using the amount of gut microorganisms associated with the cognitive function of the host as an index, and more specifically provides the following.

<12> A method for evaluating mild cognitive impairment, comprising:

(1) quantifying a microorganism in subject feces;
(2) comparing a value obtained by the quantification in step (1) with a corresponding value obtained by quantifying the microorganism in healthy subject feces; and
(3) determining that the subject is in a state of mild cognitive impairment if, as a result of the comparison in step (2), the quantitative value in the subject feces is lower than the corresponding value, wherein the microorganism is at least one microorganism selected from the group consisting of a microorganism belonging to the genus Faecalibacterium, a microorganism belonging to the genus Anaerostipes, a microorganism belonging to the genus CAG-41, a microorganism belonging to the genus Barnesiella, and a microorganism belonging to the genus Ruminococcus.

<13> A method for evaluating mild cognitive impairment, comprising:

(1) quantifying a microorganism in subject feces;
(2) comparing a value obtained by the quantification in step (1) with a corresponding value obtained by quantifying the microorganism in healthy subject feces; and
(3) determining that the subject is in a state of mild cognitive impairment if, as a result of the comparison in step (2), the quantitative value in the subject feces is higher than the corresponding value, wherein the microorganism is at least one microorganism selected from the group consisting of a microorganism belonging to the genus Prevotella.

<14> A method for evaluating Alzheimer's disease, comprising:

(1) quantifying a microorganism in subject feces;
(2) comparing a value obtained by the quantification in step (1) with a corresponding value obtained by quantifying the microorganism in healthy subject feces; and
(3) determining that the subject is suffering from Alzheimer's disease if, as a result of the comparison in step (2), the quantitative value in the subject feces is lower than the corresponding value, wherein the microorganism is at least one microorganism selected from the group consisting of a microorganism belonging to the genus Blautia, a microorganism belonging to the genus Fusicatenibacter, a microorganism belonging to the genus Ruminococcus, and a microorganism belonging to the genus Eubacterium.

<15> A method for evaluating Alzheimer's disease, comprising:

(1) quantifying a microorganism in subject feces;
(2) comparing a value obtained by the quantification in step (1) with a corresponding value obtained by quantifying the microorganism in healthy subject feces; and
(3) determining that the subject is suffering from Alzheimer's disease if, as a result of the comparison in step (2), the quantitative value in the subject feces is higher than the corresponding value, wherein the microorganism is at least one microorganism selected from the group consisting of a microorganism belonging to the genus Parabacteroides.

<16> A method for evaluating mild cognitive impairment, comprising:

(1) quantifying DNA in subject feces;
(2) comparing a value obtained by the quantification in step (1) with a corresponding value obtained by quantifying the DNA in healthy subject feces; and
(3) determining that the subject is in a state of mild cognitive impairment if, as a result of the comparison in step (2), the quantitative value in the subject feces is lower than the corresponding value, wherein the DNA is at least one of the DNAs (a) to (i).

(a) a DNA including a nucleotide sequence encoding an amino acid sequence having 40% or more homology to an amino acid sequence encoded by a nucleotide sequence set forth in SEQ ID NO: 22 or 46,
(b) a DNA including a nucleotide sequence encoding an amino acid sequence having 40% or more homology to an amino acid sequence encoded by a nucleotide sequence set forth in SEQ ID NO: 158,
(c) a DNA including a nucleotide sequence encoding an amino acid sequence having 40% or more homology to an amino acid sequence encoded by a nucleotide sequence set forth in SEQ ID NO: 204,
(d) a DNA including a nucleotide sequence encoding an amino acid sequence having 40% or more homology to an amino acid sequence encoded by a nucleotide sequence set forth in SEQ ID NO: 205,
(e) a DNA including a nucleotide sequence encoding an amino acid sequence having 40% or more homology to an amino acid sequence encoded by a nucleotide sequence set forth in SEQ ID NO: 206,
(f) a DNA including a nucleotide sequence encoding an amino acid sequence having 40% or more homology to an amino acid sequence encoded by a nucleotide sequence set forth in SEQ ID NO: 365,
(g) a DNA including a nucleotide sequence encoding an amino acid sequence having 40% or more homology to an amino acid sequence encoded by a nucleotide sequence set forth in SEQ ID NO: 414,
(h) a DNA including a nucleotide sequence encoding an amino acid sequence having 40% or more homology to an amino acid sequence encoded by a nucleotide sequence set forth in SEQ ID NO: 420, and
(i) a DNA including a nucleotide sequence encoding an amino acid sequence having 40% or more homology to an amino acid sequence encoded by a nucleotide sequence set forth in SEQ ID NO: 421.

<17> A method for evaluating mild cognitive impairment, comprising:

(1) quantifying DNA in subject feces;
(2) comparing a value obtained by the quantification in step (1) with a corresponding value obtained by quantifying the DNA in healthy subject feces; and
(3) determining that the subject is in a state of mild cognitive impairment if, as a result of the comparison in step (2), the quantitative value in the subject feces is higher than the corresponding value, wherein the DNA is a DNA containing a nucleotide sequence encoding an amino acid sequence having 40% or more homology to the amino acid sequence encoded by the nucleotide sequence set forth in SEQ ID NO: 297.

[Advantageous Effects of Invention]

According to the present invention, by administering a Faecalibacterium prausnitzii strain having a specific DNA sequence, it becomes possible to prevent or treat a deterioration in brain function, particularly a decline in cognitive function, in a subject. Furthermore, it also becomes possible to maintain or improve the subject's brain function, especially cognitive function.

Furthermore, according to the present invention, it becomes possible to diagnose dementia, mild cognitive impairment, or precursor stages thereof, using the amount of gut microorganisms as an indicator. Specifically, the present invention has identified gut microorganisms whose respective abundance correlates with mild cognitive impairment and Alzheimer's disease, and these gut microorganisms serve as an effective indicator for the diagnosis of dementia.

[Brief Description of Drawings]

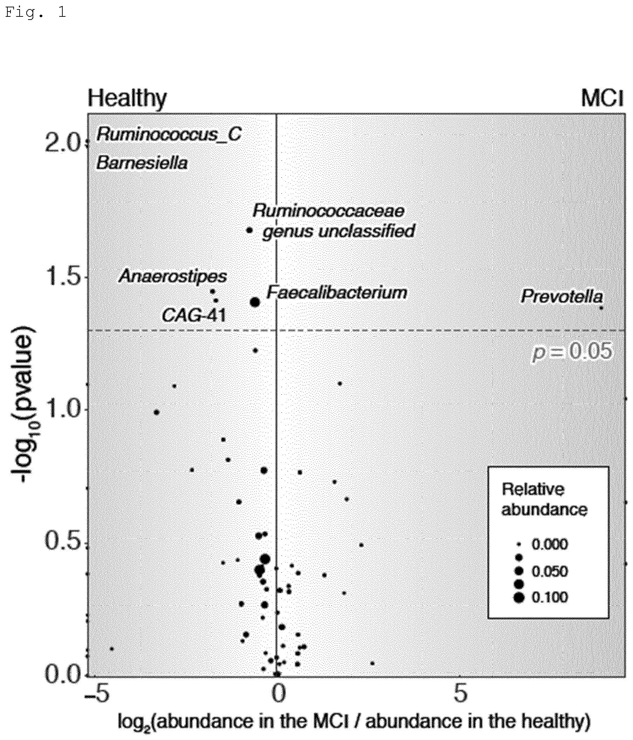

[Fig. 1] Fig. 1 is a volcano plot graph illustrating the results of comparing the gut microbiota in healthy individuals and individuals with mild cognitive impairment (MCI). In the figure, the X-axis represents the values of the ratio of the median abundance of each gut microorganism in individuals with MCI to that in healthy individuals, converted with $\log_2$, and the Y axis represents the P values calculated by the Wilcoxon rank-sum test, converted with -logic. Furthermore, the size of the circles in the figure represents the median abundance of each gut microorganism.

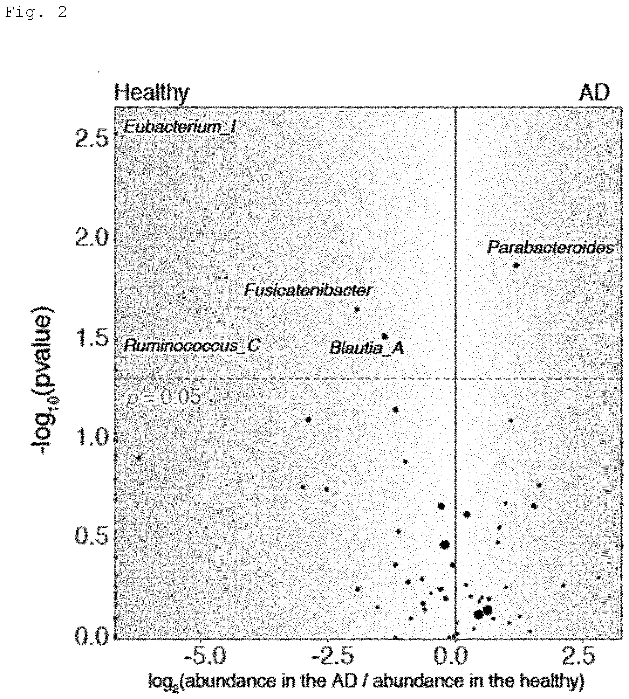

[Fig. 2] Fig. 2 is a volcano plot graph illustrating the results of comparing the gut microbiota between healthy individuals and Alzheimer's disease (AD) patients. The notation in the figure is the same as in FIG. 1, except that "individuals with MCI" is replaced with "AD patients."

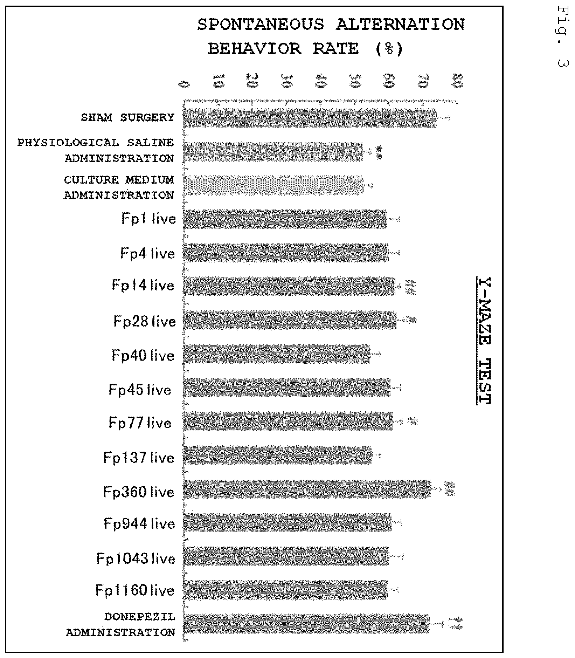

[Fig. 3] Fig. 3 is a graph illustrating the results of a study in which various strains of Faecalibacterium prausnitzii (Fp) (all live bacteria) were orally administered over a short period (11 days) to mice that had been administered with amyloid-$\beta$ into their cerebral ventricles, and the degree of improvement in learning and memory disorders in these mice was evaluated using the Y-maze test. In the figure, the horizontal axis represents the various Fp strains and control substances administered, while the vertical axis represents the spontaneous alteration rate. Each data point represents the mean value + standard error (SEM). "**" indicates that, based on the results of the Student's t-test, $P < 0.01$ compared to Control Group 1 (sham surgery). "#" and "##" respectively indicate that, based on the results of the Student's t-test, $P < 0.05$ and 0.01 compared to Control Group 3 (culture medium administration). "††" (two daggers) indicates that, based on the results of the Student's t-test, $P < 0.01$ compared to Control Group 2 (physiological saline administration).

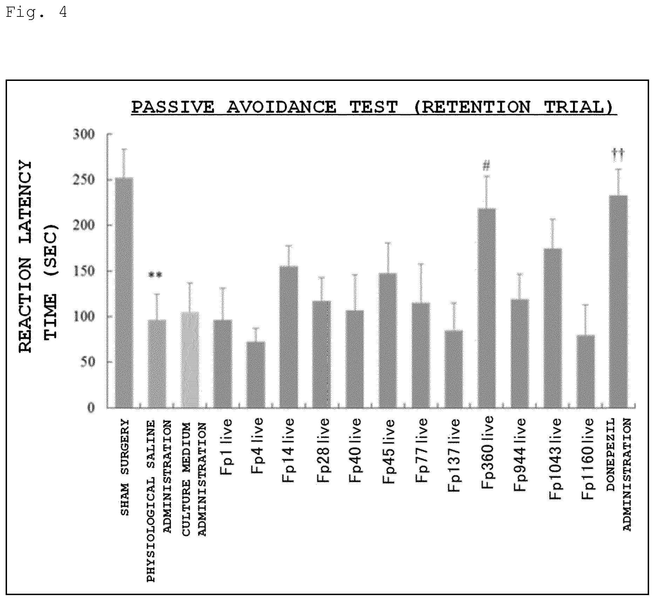

[Fig. 4] Fig. 4 is a graph illustrating the results of a study in which various Fp strains (all live bacteria) were orally administered over a short period (11 days) to mice administered with amyloid-$\beta$ into their cerebral ventricles, and the degree of improvement in learning and memory disorders in these mice was evaluated using the passive avoidance test. In the figure, the horizontal axis represents the various Fp strains and control substances administered, while the vertical axis represents the reaction latency time. Each data point represents the mean value + standard error (SEM). "**" indicates that, based on the results of the Student's t-test, $P < 0.01$ compared to Control Group 1 (sham surgery). "#" indicates that, based on the results of the Student's t-test, $P < 0.05$ compared to Control Group 3 (culture medium administration). "††" (two daggers) indicates that, based on the results of the Student's t-test, $P < 0.01$ compared to Control Group 2 (physiological saline administration).

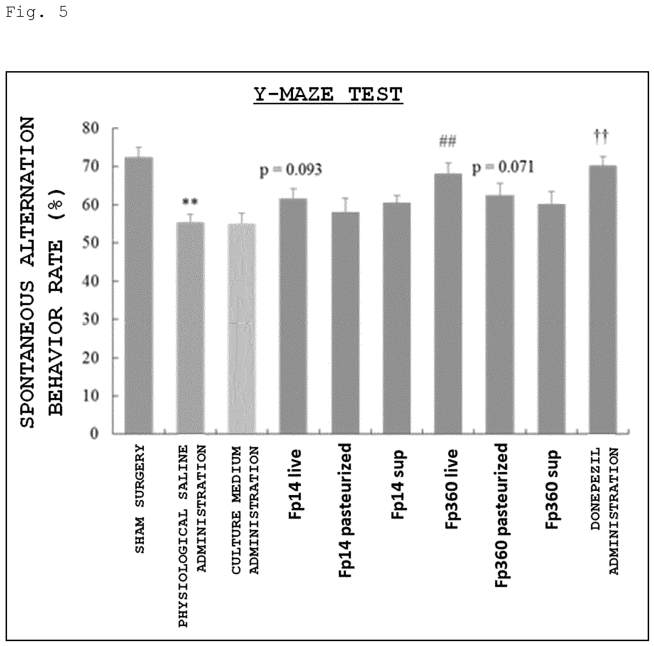

[Fig. 5] Fig. 5 is a graph illustrating the results of a study in which various Fp strains were orally administered over a short period (11 days) to mice administered with amyloid-$\beta$ into their cerebral ventricles, and the degree of improvement in learning and memory disorders in these mice was evaluated using the Y-maze test. In the figure, "live," "pasteurized," and "sup" indicate the results of administration of live bacteria, dead bacterial bodies, and culture supernatant of each bacterial strain. The notation in other figures is the same as in Fig. 3.

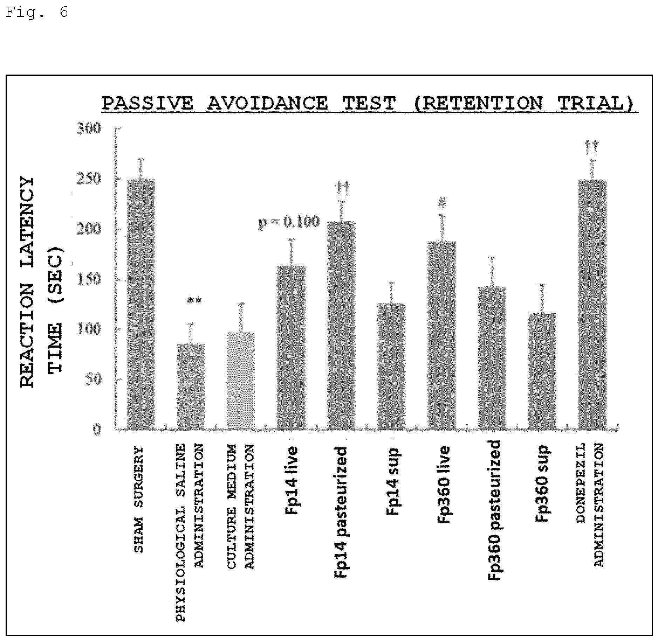

[Fig. 6] Fig. 6 is a graph illustrating the results of a study in which various Fp strains were orally administered over a short period (11 days) to mice administered with amyloid-$\beta$ into their cerebral ventricles, and the degree of improvement in learning and memory disorders in these mice was evaluated using the passive avoidance test. In the figure, "live," "pasteurized," and "sup" indicate the results of administration of live bacteria, dead bacterial bodies, and culture supernatant of each bacterial strain. The notation in other figures is the same as in Fig. 4.

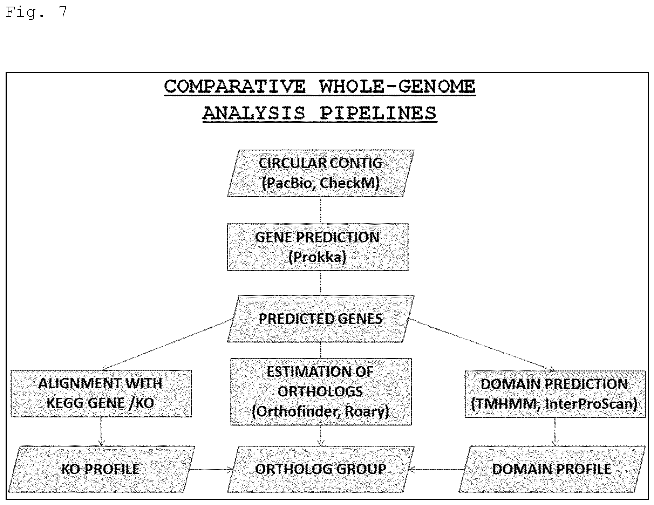

[Fig. 7] Fig. 7 is a chart illustrating an overview of the pipelines used for comparative whole-genome analysis between Fp strains.

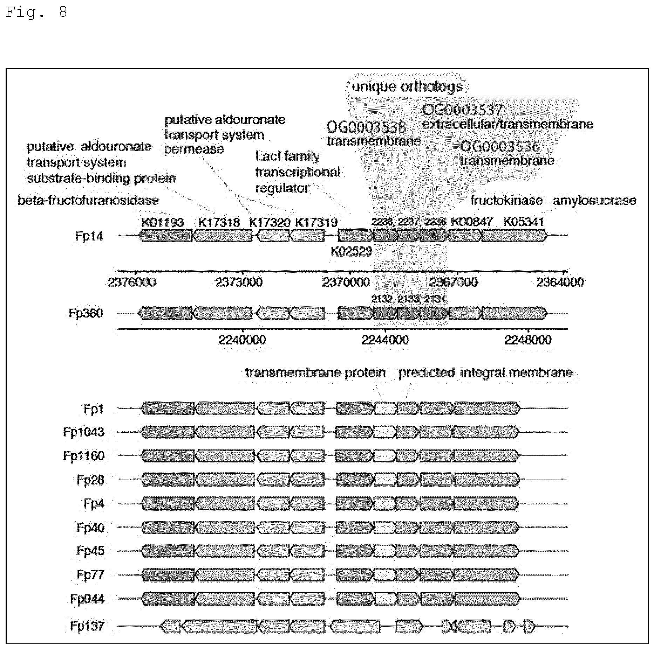

[Fig. 8] Fig. 8 is a schematic diagram illustrating that three orthologs (OG0003538, OG0003537, and OG0003536) are unique genes characterizing Fp14 and Fp360, which are arranged adjacently on the genome. In the figure, "2238," "2237," and "2236" correspond respectively to "ICBIDGAJ_02238 (SEQ ID NO: 18)," "ICBIDGAJ_02237 (SEQ ID NO: 21)," and "ICBIDGAJ_02236 (SEQ ID NO: 22)" listed in the sequence list and Table 2. "2132," "2133," and "2134" correspond respectively to "BLCPAMOF_02132 (SEQ ID NO: 55)," "BLCPAMOF_02133 (SEQ ID NO: 54)," and "BLCPAMOF_02134 (SEQ ID NO: 46)" listed in the sequence list and Table 2. "*" attached to "2236" and "2134" indicate that, according to the results of the Wilcoxon rank-sum test, the relative abundance of these orthologs is significantly lower in the MCI group compared to the healthy individual group ($P < 0.05$).

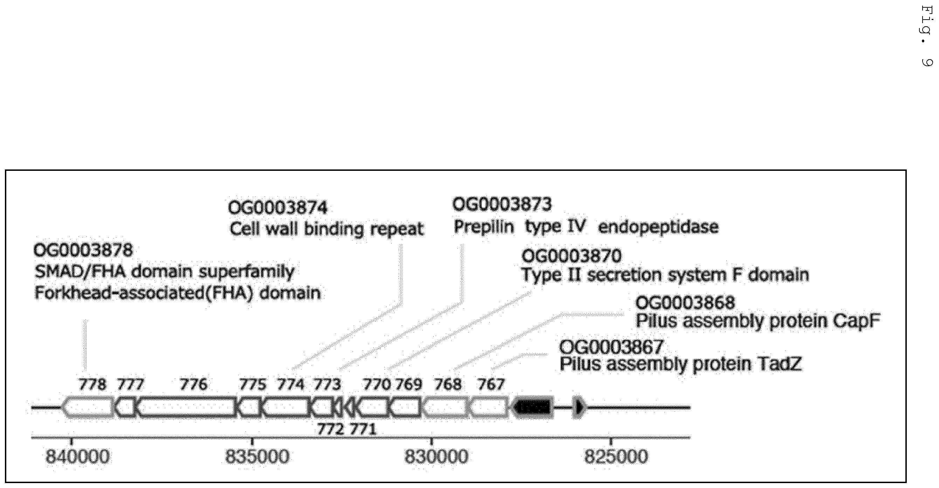

[Fig. 9] Fig. 9 is a schematic diagram illustrating that the unique twelve genes characterizing Fp14 are arranged adjacently on the genome. "778," "768," and "767" correspond to the orthologs presumed to be extracellular proteins, corresponding respectively to "ICBIDGAJ_00778 (SEQ ID NO: 162)," "ICBIDGAJ_00768 (SEQ ID NO: 198)," and "ICBIDGAJ_00767 (SEQ ID NO: 203)" listed in the sequence table. "777" to "769" are orthologs presumed to be transmembrane proteins, corresponding respectively to "ICBIDGAJ_00777 (SEQ ID NO: 192)," "ICBIDGAJ_00776 (SEQ ID NO: 191)," "ICBIDGAJ_00775 (SEQ ID NO: 190)," "ICBIDGAJ_00774 (SEQ ID NO: 189)," "ICBIDGAJ_00773 (SEQ ID NO: 188)," "ICBIDGAJ_00772 (SEQ ID NO: 187)," "ICBIDGAJ_00771 (SEQ ID NO: 186)," "ICBIDGAJ_00770 (SEQ ID NO: 185)," and "ICBIDGAJ_00769 (SEQ ID NO: 59)" listed in the sequence table. Note that In the figure, the two blackened orthologs without numbers around 825000 are both non-specific orthologs of Fp14, presumed to be extracellular proteins.

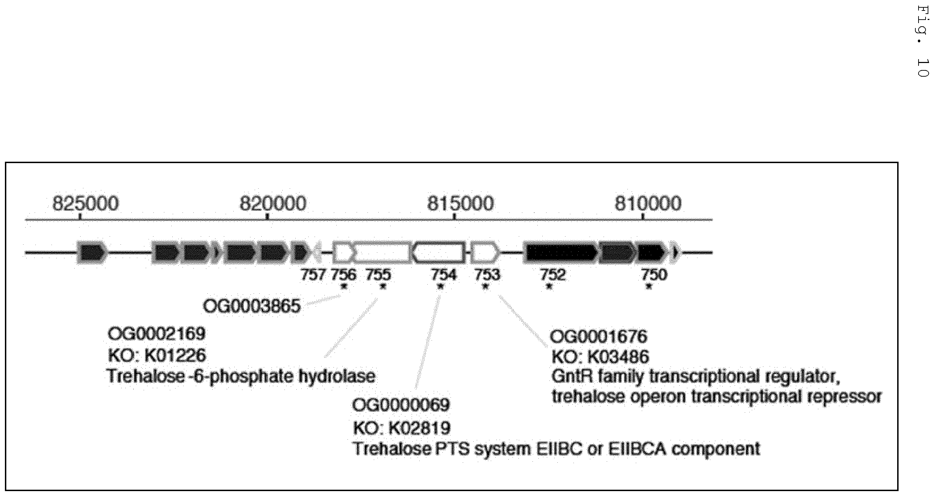

[Fig. 10] Fig. 10 is a schematic diagram illustrating that the unique five genes characterizing Fp14 are arranged adjacently on the genome. In the figure, "757" and "756" correspond respectively to "ICBIDGAJ_00757 (SEQ ID NO: 159)" and "ICBIDGAJ_00756 (SEQ ID NO: 158)" listed in the sequence list. The trehalose-related KOs "755," "754," and "753" correspond respectively to "ICBIDGAJ_00755 (SEQ ID NO: 205)," "ICBIDGAJ_00754 (SEQ ID NO: 204)," and "ICBIDGAJ_00753 (SEQ ID NO: 206)" listed in the sequence list. Note that "757" is an ortholog presumed to be an intracellular protein, "756," "755," and "753" are ortholog each presumed to be an extracellular protein, and "754" is an ortholog presumed to be a transmembrane protein. All other blackened orthologs are non-specific Fp14 orthologs. "*" attached to "750" to "756" indicate that, according to the results of the Wilcoxon rank-sum test, the relative abundance of these orthologs is significantly lower in the MCI group compared to the healthy individual group ($P < 0.05$).

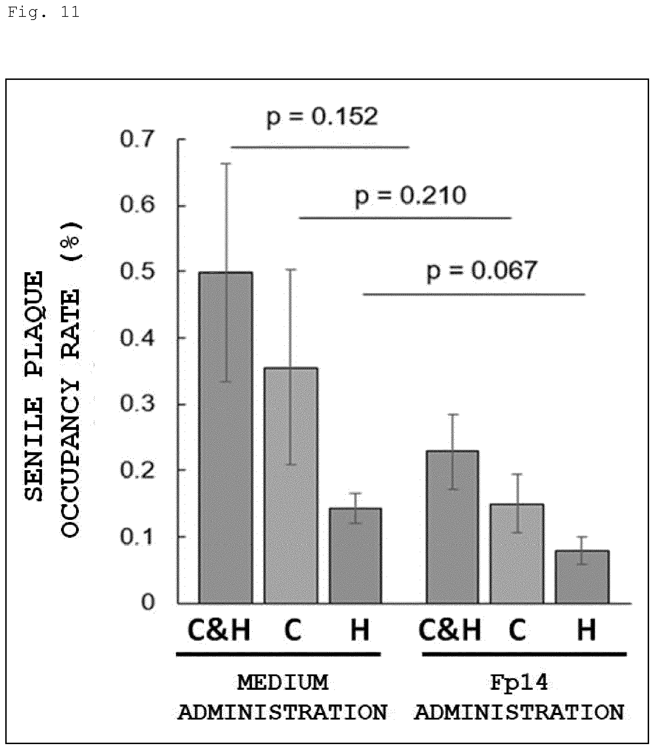

[Fig. 11] Fig. 11 is a graph indicating the senile plaque occupancy rate (%) in the brain tissue of AD model mice that had been orally administered with Fp14 over a long period of time (13 months). In the figure, the horizontal axis represents the substance that was orally administered, and "C&H," "C, " and "H" each represent the results of analyzing the "cerebral cortex and hippocampus," "cerebral cortex," and "hippocampus," respectively. Each data point represents the mean value + standard error (SEM). The p-values noted in the figure were calculated using Student's t-test.

[Description of Embodiments]

(Faecalibacterium prausnitzii)

As illustrated in the examples described later, it has been made clear by the present inventors that administering a strain of Faecalibacterium prausnitzii, which has at least one of the DNAs according to the present invention, improves the deterioration in brain function in the subject. Therefore, the present invention relates to Faecalibacterium prausnitzii having a specific DNA sequence.

"Faecalibacterium prausnitzii" generally refers to an anaerobic, gram-negative bacillus that belongs to the genus Faecalibacterium, under the Oscillospiraceae family, Eubacteriales order, Clostridia class, and Firmicutes phylum. This microorganism (bacterium) does not form spores and has the characteristic of producing butyric acid from acetic acid. Moreover, Faecalibacterium prausnitzii may also include microorganisms identified as identical or substantially identical to known Faecalibacterium prausnitzii based on one comparison results of their characteristics, such as morphological characteristics (such as colony shape and cell shape), physiological and biochemical characteristics such as sugar assimilation, growth temperature, and optimal pH), and chemotaxonomic characteristics (such as fatty acid composition of the bacterial body). Additionally, microorganisms identified as identical or substantially identical based on the analysis of the nucleotide sequence of the 16S rRNA gene may also be included.

More specifically, a microorganism identified as identical or substantially identical to the known Faecalibacterium prausnitzii refers to a microorganism of the same genus, whose 16S rRNA gene base sequence has 97% or more, preferably 98% or more, even more preferably 99% or more, and even more most preferably 100% homology with the base sequence of the 16S rRNA gene of any microorganism of the known Faecalibacterium prausnitzii, and also preferably has the same microbiological properties (bacteriological properties) as any of the known Faecalibacterium prausnitzii.

Furthermore, as long as the effects of the present invention are not impaired, it may be a microorganism that has been bred through mutation processing, genetic recombination, or the selection of natural mutant strains from each microorganism of the well-known Faecalibacterium prausnitzii. Such "mutant strains" refer to specific microorganisms (bacterial strains) that have been mutated by methods well-known to those skilled in the art, without affecting their properties, those that have been bred through the selection of natural mutant strains or the like, or those that can be confirmed by those skilled in the art to be equivalent to them.

Additionally, the known Faecalibacterium prausnitzii is not limited to the strain itself deposited or registered under this bacterial strain name at a predetermined institution (hereinafter referred to as "deposited strain"), and also includes strains that are substantially equivalent to it (also referred to as "derived strains" or "induced strains").

Regarding bacterial strains, a "strain substantially equivalent to a deposited strain" means a strain that belongs to the same species as the deposited strain and is capable of exhibiting the effects of the present invention. A strain substantially equivalent to a deposited strain may be, for example, a derived strain that has the deposited strain as a parent strain. Derived strains include strains bred from the deposited strain and strains that arise naturally from the deposited strain.

Substantially identical bacterial strains and derived strains include the following strains.

(1) Bacterial strains determined to be the same bacterial strain by the RAPD method (Randomly Amplified Polymorphic DNA) and the PFGE method (Pulsed-field gel electrophoresis) method (described in Probiotics in food/Health and nutritional properties and guidelines for evaluation 85, Page 43)

(2) Bacterial strains that have only genes derived from the deposited bacterial strain, have no foreign genes, and have a DNA homology of 97% or more (preferably 98% or more)

(3) Strains bred from the bacterial strain (including genetic engineering modifications, mutations, and spontaneous mutations) and strains having the same traits.

The "Faecalibacterium prausnitzii according to the present invention" is a microorganism having at least one DNA in the DNA group according to the present invention below in the above-mentioned general Faecalibacterium prausnitzii.

As demonstrated in the examples described later, as sequences corresponding to 28 orthologs unique to Fp14 and Fp360, which were found to have an improvement effect on the deterioration in brain function, DNA sequences set forth in SEQ ID NOs: 1 to 57 and 422 have been extracted. Therefore, the DNA group according to the present invention includes the group composed of the DNAs described in the following (1) to (28).

(1) a DNA including a nucleotide sequence encoding an amino acid sequence having 40% or more homology to an amino acid sequence encoded by a nucleotide sequence set forth in SEQ ID NO: 22 or 46,

(2) a DNA including a nucleotide sequence encoding an amino acid sequence having 40% or more homology to an amino acid sequence encoded by a nucleotide sequence set forth in SEQ ID NO: 21 or 54,

(3) a DNA including a nucleotide sequence encoding an amino acid sequence having 40% or more homology to an amino acid sequence encoded by a nucleotide sequence set forth in SEQ ID NO: 18 or 55,

(4) a DNA including a nucleotide sequence encoding an amino acid sequence having 40% or more homology to an amino acid sequence encoded by a nucleotide sequence set forth in SEQ ID NO: 9 or 30,

(5) a DNA including a nucleotide sequence encoding an amino acid sequence having 40% or more homology to an amino acid sequence encoded by a nucleotide sequence set forth in SEQ ID NO: 19 or 33,

(6) a DNA including a nucleotide sequence encoding an amino acid sequence having 40% or more homology to an amino acid sequence encoded by a nucleotide sequence set forth in SEQ ID NO: 17 or 34,

(7) a DNA including a nucleotide sequence encoding an amino acid sequence having 40% or more homology to an amino acid sequence encoded by a nucleotide sequence set forth in SEQ ID NO: 3 or 43,

(8) a DNA including a nucleotide sequence encoding an amino acid sequence having 40% or more homology to an amino acid sequence encoded by a nucleotide sequence set forth in SEQ ID NO: 6 or 53,

(9) a DNA including a nucleotide sequence encoding an amino acid sequence having 40% or more homology to an amino acid sequence encoded by a nucleotide sequence set forth in SEQ ID NO: 7 or 35,

(10) a DNA including a nucleotide sequence encoding an amino acid sequence having 40% or more homology to an amino acid sequence encoded by a nucleotide sequence set forth in SEQ ID NO: 2 or 39,

(11) a DNA including a nucleotide sequence encoding an amino acid sequence having 40% or more homology to an amino acid sequence encoded by a nucleotide sequence set forth in SEQ ID NO: 25 or 41,

(12) a DNA including a nucleotide sequence encoding an amino acid sequence having 40% or more homology to an amino acid sequence encoded by a nucleotide sequence set forth in SEQ ID NO: 23 or 50,

(13) a DNA including a nucleotide sequence encoding an amino acid sequence having 40% or more homology to an amino acid sequence encoded by a nucleotide sequence set forth in SEQ ID NO: 24 or 45,

(14) a DNA including a nucleotide sequence encoding an amino acid sequence having 40% or more homology to an amino acid sequence encoded by a nucleotide sequence set forth in SEQ ID NO: 26 or 48,

(15) a DNA including a nucleotide sequence encoding an amino acid sequence having 40% or more homology to an amino acid sequence encoded by a nucleotide sequence set forth in SEQ ID NO: 8 or 40,

(16) a DNA including a nucleotide sequence encoding an amino acid sequence having 40% or more homology to an amino acid sequence encoded by a nucleotide sequence set forth in SEQ ID NO: 4 or 37,

(17) a DNA including a nucleotide sequence encoding an amino acid sequence having 40% or more homology to an amino acid sequence encoded by a nucleotide sequence set forth in SEQ ID NO: 13 or 38,

(18) a DNA including a nucleotide sequence encoding an amino acid sequence having 40% or more homology to an amino acid sequence encoded by a nucleotide sequence set forth in SEQ ID NO: 16 or 56,

(19) a DNA including a nucleotide sequence encoding an amino acid sequence having 40% or more homology to an amino acid sequence encoded by a nucleotide sequence set forth in SEQ ID NO: 14 or 57,

(20) a DNA including a nucleotide sequence encoding an amino acid sequence having 40% or more homology to an amino acid sequence encoded by a nucleotide sequence set forth in SEQ ID NO: 27 or 36,

(21) a DNA including a nucleotide sequence encoding an amino acid sequence having 40% or more homology to an amino acid sequence encoded by a nucleotide sequence set forth in SEQ ID NO: 12 or 42,

(22) a DNA including a nucleotide sequence encoding an amino acid sequence having 40% or more homology to an amino acid sequence encoded by a nucleotide sequence set forth in SEQ ID NO: 15, 31, or 49,

(23) a DNA including a nucleotide sequence encoding an amino acid sequence having 40% or more homology to an amino acid sequence encoded by a nucleotide sequence set forth in SEQ ID NO: 5, 52, or 51,

(24) a DNA including a nucleotide sequence encoding an amino acid sequence having 40% or more homology to an amino acid sequence encoded by a nucleotide sequence set forth in SEQ ID NO: 20 or 44,

(25) a DNA including a nucleotide sequence encoding an amino acid sequence having 40% or more homology to an amino acid sequence encoded by a nucleotide sequence set forth in SEQ ID NO: 1 or 32,

(26) a DNA including a nucleotide sequence encoding an amino acid sequence having 40% or more homology to an amino acid sequence encoded by a nucleotide sequence set forth in SEQ ID NO: 11 or 29,

(27) a DNA including a nucleotide sequence encoding an amino acid sequence having 40% or more homology to an amino acid sequence encoded by a nucleotide sequence set forth in SEQ ID NO: 10 or 47, and

(28) a DNA including a nucleotide sequence encoding an amino acid sequence having 40% or more homology to an amino acid sequence encoded by a nucleotide sequence set forth in SEQ ID NO: 28 or 422.

Among the DNAs listed in (1) to (28), the Faecalibacterium prausnitzii according to the present invention is a microorganism that has preferably 2 or more (for example, 3 or more, 4 or more), more preferably 5 or more (for example, 6 or more, 7 or more, 8 or more, 9 or more), further preferably 10 or more (for example, 11 or more, 12 or more, 13 or more, 14 or more), more preferably 15 or more (for example, 16 or more, 17 or more, 18 or more, 19 or more), further preferably 20 or more (for example, 21 or more, 22 or more, 23 or more, 24 or more), more preferably 25 or more (for example, 26 or more, 27 or more), and particularly preferably 28 DNAs (that is, Faecalibacterium prausnitzii that has 28 DNAs listed in (1) to (28)).

Also, the Faecalibacterium prausnitzii according to the present invention is preferably a microorganism having at least one of the DNAs (1) to (3), more preferably a microorganism having at least two of the DNAs (1) to (3), and further preferably a microorganism having three of the DNAs (1) to (3).

Furthermore, the Faecalibacterium prausnitzii according to the present invention is preferably a microorganism having at least the DNA described in (1).

Moreover, in addition to at least one of the DNAs (1) to (28), at least one of the DNAs (1) to (3), or the DNA described in (1), the Faecalibacterium prausnitzii according to the present invention may contain at least one DNA selected from a group of 150 DNAs each containing a nucleotide sequence according to SEQ ID NOs: 58 to 207 and/or a group of 214 DNAs each containing a nucleotide sequence according to SEQ ID NOs: 208 to 421, described later.

As demonstrated in the examples described later, as sequences corresponding to 150 orthologs unique only to Fp14, which were found to have an improvement effect on the deterioration in brain function, DNA sequences set forth in SEQ ID NOs: 58 to 207 have been extracted. Therefore, the DNA group according to the present invention includes the following DNA group.

A group of 150 DNAs each containing a nucleotide sequence according to SEQ ID NOs: 58 to 207 (Note that the nucleotide sequence according to SEQ ID NO: N means a nucleotide sequence encoding an amino acid sequence having 40% or more homology to the amino acid sequence encoded by the nucleotide sequence set forth in SEQ ID NO: N. "N" here is any of 58 to 207.).

Of the group of 150 DNAs each containing a nucleotide sequence according to SEQ ID NOs: 58 to 207, the Faecalibacterium prausnitzii according to the present invention is a microorganism that has preferably 2 or more (for example, 3 or more, 4 or more), more preferably 5 or more (for example, 6 or more, 7 or more, 8 or more, 9 or more), further preferably 10 or more, more preferably 20 or more, further preferably 30 or more, more preferably 40 or more, further preferably 50 or more, more preferably 60 or more, further preferably 70 or more, more preferably 80 or more, further preferably 90 or more, more preferably 100 or more, further preferably 110 or more, more preferably 120 or more, further preferably 130 or more, more preferably 140 or more (for example, 141 or more, 142 or more, 143 or more, 144 or more, 145 or more, 146 or more, 147 or more, 148 or more, 149 or more), and particularly preferably 150 DNAs.

Also, the Faecalibacterium prausnitzii according to the present invention is a microorganism that has at least one DNA, preferably two or more DNAs, more preferably three or more DNAs, and further preferably four DNAs, selected from the group consisting of four DNAs each including the nucleotide sequences according to SEQ ID NOs: 158 and 204 to 206.

Moreover, in addition to at least one DNA selected from a group of 150 DNAs each containing a nucleotide sequence according to SEQ ID NOs: 58 to 207 or a group of 4 DNAs each containing a nucleotide sequence according to SEQ ID NOs: 158 and 204 to 206, the Faecalibacterium prausnitzii according to the present invention may contain at least one of the DNAs (1) to (28), at least one of the DNAs (1) to (3), or the DNA described in (1).

As demonstrated in the examples described later, as sequences corresponding to 214 orthologs unique only to Fp360, which were found to have an improvement effect on the deterioration in brain function, DNA sequences set forth in SEQ ID NOs: 208 to 421 have been extracted. Therefore, the DNA group according to the present invention includes the following DNA group.

A group of 214 DNAs each containing a nucleotide sequence according to SEQ ID NOs: 208 to 421 (Note that the nucleotide sequence according to SEQ ID NO: N means a nucleotide sequence encoding an amino acid sequence having 40% or more homology to the amino acid sequence encoded by the nucleotide sequence set forth in SEQ ID NO: N. "N" here is any of 208 to 421.).

Of the group of 214 DNAs each containing a nucleotide sequence according to SEQ ID NOs: 208 to 421, the Faecalibacterium prausnitzii according to the present invention is a microorganism that has preferably 2 or more (for example, 3 or more, 4 or more), more preferably 5 or more (for example, 6 or more, 7 or more, 8 or more, 9 or more), further preferably 10 or more, more preferably 20 or more, further preferably 30 or more, more preferably 40 or more, further preferably 50 or more, more preferably 60 or more, further preferably 70 or more, more preferably 80 or more, further preferably 90 or more, more preferably 100 or more, further preferably 110 or more, more preferably 120 or more, further preferably 130 or more, more preferably 140 or more, further preferably 150 or more, more preferably 160 or more, further preferably 170 or more, more preferably 180 or more, further preferably 190 or more, more preferably 200 or more (for example, 201 or more, 202 or more, 203 or more, 204 or more), further preferably 205 or more (for example, 206 or more, 207 or more, 208 or more, 209 or more), more preferably 210 or more (for example, 211 or more, 212 or more, 213 or more), and particularly preferably 214 DNAs.

Also, the Faecalibacterium prausnitzii according to the present invention is a microorganism that has at least one DNA, preferably two or more DNAs, more preferably three or more DNAs, and further preferably four DNAs, selected from the group consisting of four DNAs each including the nucleotide sequences according to SEQ ID NOs: 365, 414, 420, and 421.

Moreover, in addition to at least one DNA selected from a group of 214 DNAs each containing a nucleotide sequence according to SEQ ID NOs: 208 to 421 or a group of 4 DNAs each containing a nucleotide sequence according to SEQ ID NOs: 365, 414, 420, and 421, the Faecalibacterium prausnitzii according to the present invention may contain at least one of the DNAs (1) to (28), at least one of the DNAs (1) to (3), or the DNA described in (1).

Note that in the present invention, in addition to the nucleotide sequence encoding an amino acid sequence having a 40% or more homology to the amino acid sequence encoded by the nucleotide sequence set forth in SEQ ID NO: N, the DNA according to the present invention may take the form of a DNA that contains a nucleotide sequence encoding an amino acid sequence, where one or more amino acids are substituted, deleted, added, and/or inserted, in the amino acid sequence encoded by the nucleotide sequence set forth in SEQ ID NO: N, or a DNA that hybridizes under stringent conditions with the DNA composed of the nucleotide sequence set forth in SEQ ID NO: N (N here is at least one number from 1 to 422).

"Homology" can mean similarity or identify. In addition, "homology" may specifically imply identify. The homology of amino acid sequences can be determined using alignment programs such as BLAST (Basic Local Alignment Search Tool at the National Center for Biological Information). For example, the homology of amino acid sequences can refer to the homology between amino acid sequences calculated using blastp, and more specifically to the homology between amino acid sequences calculated using blastp with default parameters (that is, using the initially set parameters).

The "homology" of amino acid sequences according to the present invention is 40% or more, preferably 50% or more, more preferably 60% or more, further preferably 70% or more, more preferably 80% or more, further preferably 85% or more (for example, 86% or more, 87% or more, 88% or more, 89% or more), more preferably 90% or more (for example, 910 or more, 92% or more, 93% or more, 94% or more), further preferably 95% or more (for example, 96% or more, 97% or more, 98% or more, 99% or more), and particularly preferably 100%.

In the present invention, the number of amino acids to be substituted and the like is not particularly limited, as long as the polypeptide containing the amino acid sequence encoded by the nucleotide sequence set forth in each sequence number does not lose its function or activity due to such substitution, and examples thereof include 1000 amino acids or less (more specifically, 900 amino acids or less, 800 amino acids or less, 700 amino acids or less, and 600 amino acids or less), 500 amino acids or less (more specifically, 400 amino acids or less, 300 amino acids or less, and 200 amino acids or less), 100 amino acids or less (more specifically, 90 amino acids or less, 80 amino acids or less, 70 amino acids or less, and 60 amino acids or less), 50 amino acids or less (more specifically, 40 amino acids or less, 30 amino acids or less, and 20 amino acids or less), and 10 amino acids or less (more specifically, 9 amino acids or less, 8 amino acids or less, 7 amino acids or less, 6 amino acids or less, 5 amino acids or less, 4 amino acids or less, 3 amino acids or less, 2 amino acids or less, and 1 amino acid).

Substitution or the like of amino acids, preferably, is a conservative substitution. A "conservative substitution" refers to the substitution with another amino acid residue that has a chemically similar side chain. Groups of amino acid residues having chemically similar side chains are well known in the art to which the present invention belongs. For example, they can be categorized as acidic amino acids (aspartic acid and glutamic acid), basic amino acids (lysine, arginine, and histidine), and neutral amino acids, namely amino acids with hydrocarbon chains (glycine, alanine, valine, leucine, isoleucine, and proline), amino acids with hydroxyl groups (serine and threonine), sulfur-containing amino acids (cysteine and methionine), amino acids with amide groups (asparagine and glutamine), amino acids with an imino group (proline), and amino acids with aromatic groups (phenylalanine, tyrosine, and tryptophan).

The "stringent conditions" are, for example, hybridization conditions of about "1 X SSC, 0.1% SDS, 37°C," and more stringent conditions include hybridization conditions of about "0.5 X SSC, 0.1% SDS, 42°C," and further more stringent conditions include hybridization conditions of about "0.2 X SSC, 0.1% SDS, 65°C."

The Faecalibacterium prausnitzii according to the present invention can be appropriately prepared by those skilled in the art using known methods. For example, it can be prepared by culturing digestive tract contents (such as feces) from humans, non-human mammals (such as cloven-hoofed animals including pigs and cattle, rodents such as mice), poultry, and cockroaches, under anaerobic conditions in known media (for example, YCFA medium, EG medium, or LYBHI medium) as illustrated in the examples described later.

Furthermore, as demonstrated in the examples described later, Fp14 and Fp360 are particularly preferable embodiments of the Faecalibacterium prausnitzii according to the present invention, and Fp14 was deposited on March 6, 2020, at NITE Patent Microorganisms Depositary, National Institute of Technology and Evaluation (NITE, Room 122, 2-5-8 Kazusa-kamatari, Kisarazu City, Chiba Prefecture, zip code 292-0818) (identification: Fp_0014 and accession number: NITE BP-03169). Fp360 was deposited on April 7, 2020, at NITE Patent Microorganisms Depositary, National Institute of Technology and Evaluation (identification: Fp_0360 and accession number: NITE BP-03196). Therefore, these bacterial strains can be obtained by applying to the aforementioned deposit center.

Note that, as described above, the Faecalibacterium prausnitzii according to the present invention may include microorganisms identified as identical or substantially identical to Fp14 and/or Fp360, in other words, mutant strains, derived strains, and induced strains thereof without being specified by the deposited strains specified by the accession numbers above, as long as the effects of the present invention are not impaired. More specifically, they can be microorganisms of the same genus as Fp14 and Fp360, having a base sequence of the 16S rRNA gene with a 97% or more, preferably 98% or more, more preferably 99% or more, and more preferably 1000 or more homology with the base sequence of the 16S rRNA gene of Fp14 and/or Fp360, and preferably, having the same microbiological properties (bacteriological properties) as any of these microorganisms.

In addition, similarly, in the present invention, they may be mutant strains, derived strains, or induced strains of Faecalibacterium prausnitzii having at least one DNA in the DNA group according to the present invention described above.

In addition, the Faecalibacterium prausnitzii according to the present invention has therapeutic and preventative effects against the deterioration in brain function, and also has the maintenance action and improvement action for brain function. Whether this microorganism has such effects or actions can be evaluated, for example, through a behavioral test using non-human animals with impaired brain function, as demonstrated in the examples described later. More specifically, when the microorganism is administered to non-human animals with impaired brain function (for example, Alzheimer's disease model animals), and an improvement in brain function is observed in comparison to the case not given the same, it can be judged that the microorganism has the aforementioned effects and actions. Note that the evaluation of brain function can be conducted by selecting known behavioral tests suited to the brain function that those skilled in the art wish to evaluate.

The Faecalibacterium prausnitzii according to the present invention preferably has the action of inhibiting the accumulation of amyloid-β in the brain. The "amyloid-β" according to the present invention refers to a peptide composed of 36 to 43 amino acids, which is produced by cleavage of a membrane protein, the amyloid β precursor protein (APP), by β-secretase and γ-secretase. It is preferably a peptide composed of 42 amino acids (amyloid-β42) or 40 amino acids (amyloid-β40), and more preferably amyloid-β42. In addition, the form of amyloid-β to be accumulated is not particularly limited, it may be a monomer, may form an oligomer (amyloid-β oligomer), may be fibrillized (amyloid fibrils), or may be a mixture of these.

Since amyloid-β is thought to be involved in the onset and progression of Alzheimer's disease, Faecalibacterium prausnitzii, which has the action of inhibiting its accumulation, can have excellent preventive and other effects against the disease. Whether this microorganism has such actions can be evaluated, for example, through a test using Alzheimer's disease model animals, as demonstrated in the examples described later. More specifically, when the microorganism is administered to Alzheimer's disease model animals, and the amount of amyloid-β in the brain is reduced in comparison to the case not given the same, it can be judged that this microorganism has the action of inhibiting the accumulation of amyloid-β in the brain.

<Composition for preventing or treating deterioration in brain function, or for maintaining or improving brain function>

As shown in the examples described later, the administration of Faecalibacterium prausnitzii according to the present invention inhibits the deterioration in brain function in the subject. Therefore, the present invention provides a composition for preventing or treating deterioration in brain function, or for maintaining or improving brain function in a subject, including Faecalibacterium prausnitzii according to the present invention, a component of the microorganism, or a culture of the microorganism as an active ingredient.

Faecalibacterium prausnitzii contained as an active ingredient in the composition of the present invention is as described above, and may be live bacteria, dead bacterial bodies, or both live bacteria and dead bacterial bodies. Examples of "dead bacterial bodies" include heat-treated, calcined, steamed, irradiated (such as γ-ray), enzyme-treated, drug (such as antibiotics)-treated, chemically (such as formalin) treated, and ultrasonic-treated ones. Also, the form of Faecalibacterium prausnitzii is not particularly limited and can be either liquid or solid, but a dry form (such as powdered bacteria) is preferable from the viewpoint of ease of handling during production and storage. Dried products can be prepared by drying a suspension in which the bacterial body is dispersed in a solvent (such as water). The drying method is not particularly limited, and examples thereof include spray drying and freeze drying. The sterilization method is not particularly limited, and examples thereof include retort sterilization, UHT sterilization, air sterilization, pressure sterilization, high-pressure steam sterilization, dry heat sterilization, flowing steam disinfection, microwave sterilization, electron beam sterilization, high-frequency sterilization, radiation sterilization, ultraviolet sterilization, ethylene oxide gas sterilization, hydrogen peroxide plasma sterilization, and chemical sterilization methods (such as alcohol sterilization, formalin fixation, and electrolyzed water treatment). Additionally, depending on the case, treatments such as surfactant treatment, grinding or pulverization processing, enzyme treatment, and fractionation may be conducted before and after sterilization by heating or the like, or before and after drying, and the composition of the present invention may contain products obtained through these treatments.

The Faecalibacterium prausnitzii contained as an active ingredient in the composition of the present invention may not only be the aforementioned bacterial body, but also the constituent substances of the microorganism, that is, the components of the microorganism itself (so-called bacterial body components. For example, they may be amino acids, peptides, nucleic acids, sugars, lipids, vitamins, hormones, and complexes thereof, and even complexes of these with phosphorus-sulfur metal elements, and the like, constituting the microorganism).

Furthermore, the active ingredient contained in the composition of the present invention may be a culture of Faecalibacterium prausnitzii according to the present invention. The culture may contain the microorganism (such as a culture fluid containing the proliferated microorganism or solid media), and the form of the culture is not particularly limited, and may be either liquid or solid, but the form is preferably a dried form (such as dried culture) from the viewpoint of ease of handling during production and storage. Note that such dried cultures and the like can be appropriately prepared by those skilled in the art using the aforementioned drying methods.

The composition of the present invention can be in the form of a pharmaceutical composition (such as drugs and quasi-drugs), a food composition (such as food and/or beverage and animal feed), or a reagent used in model animal experiments and the like.

The composition in the present invention, depending on the form of the Faecalibacterium prausnitzii as an active ingredient, can be formulated using known pharmaceutical methods (such as live bacteria, dead bacterial bodies, and bacterial body components). For instance, it can be used orally or parenterally as capsules, tablets, pills, liquids, powders, granules, fine granules, film-coated agents, pellets, troches, sublingual agents, chewable agents, buccal agents, paste agents, syrup agents, suspensions, elixir agents, emulsion agents, liniments, ointments, hard ointments, cataplasms, transdermal systems, lotions, inhalants, aerosols, injections, suppositories, and the like. As shown in the examples described later, the present invention can be orally taken non-invasively and conveniently. That is, the preferable method of intake for the composition of the present invention is oral intake.

In formulating these preparations, it is possible to appropriately combine with carriers that are acceptable pharmacologically or as food and/or beverage, specifically, physiological saline, sterile water, culture media, vegetable oils, solvents, excipients, bases, emulsifiers, suspensions, surfactants, stabilizers, flavoring agents, aromatics, vehicles, antiseptics, binding agents, diluents, tonicity adjusting agents, analgesics, bulking agents, disintegrators, buffer agents, coating agents, lubricants, colorants, sweeteners, thickening agents, flavor modifiers, solubilizers, or other additives.

When using the composition of the present invention as a pharmaceutical composition, it may be used in combination with known substances used for improving conditions such as brain function deterioration and the like. Such known substances include, for example, dementia drugs, more specifically, cholinesterase inhibitors (such as donepezil), acetylcholinesterase inhibitors (such as galantamine), glutamic acid NMDA receptor inhibitors (such as memantine), agents for inhibiting the production of amyloid-β (such as γ-secretase modulator (GSM), γ-secretase inhibitor (GSI), and non-steroidal anti-inflammatory drugs), agents for inhibiting the aggregation of amyloid-β (such as curcumin, polysulfide compounds, and clioquinol), agents for inhibiting the aggregation of tau (such as aminothienopyridazines, cyanine dyes, and methylene blue), and neuroprotective agents (such as dimebon) .

When used as a food composition, the composition of the present invention may be found in various forms such as health foods, functional foods, foods for specified health uses, foods with nutrient function claims, foods with functional claims, dietary supplements, foods for patients, or animal feeds. Note that functional foods are typically classified into four categories based on their mechanism of action, probiotics, biogenics, prebiotics, and synbiotics, and in the present invention, they can take the form of probiotics, biogenics, synbiotics, or prebiotics.

The food and/or beverage of the present invention can be consumed not only as the aforementioned compositions, but also in various other foods and/or beverages. Specific examples of these foods and/or beverages include liquid foods such as functional beverages, drink preparations, jelly beverages, refreshing beverages, dairy beverages, tea beverages, alcoholic beverages, and soups; fermented foods and fermented beverages such as yogurt and drinkable yogurt; products containing fats such as edible oils, dressings, mayonnaise, and margarine; carbohydrate-containing foods such as rice food products, noodles, and bread; processed animal foods such as ham and sausages; processed fishery foods such as steamed fish paste, dried fish, and salted seafood; processed vegetable foods such as pickles; semi-solid foods such as jellies; fermented foods such as fermented soybean paste; various types of confectionery such as Western confectionery products, Japanese confectionery products, candies, gums, gummies, cold desserts, and frozen desserts; retort pouch products such as curry, thick starchy sauces, and Chinese soups; instant foods and microwavable foods such as instant soups and instant soybean soup. Furthermore, health foods and/or beverages prepared in forms such as powders, granules, tablets, capsules, liquids, pastes, or jellies can also be mentioned. The production of the food and/or beverage of the present invention can be implemented using production techniques known in the art. In these foods and/or beverages, one or more components effective in improving deterioration in brain function and the like (such as other gut microorganisms and components effective for the proliferation of Faecalibacterium prausnitzii in the intestinal tract and the like) may be added. In addition, by combining with other components that have functions apart from the above improvement and the like, or with other functional foods, they may also be used as multifunctional foods and/or beverages.

Note that the "other gut microorganisms" above are not particularly limited, but include microorganisms belonging to the genus Faecalibacterium, microorganisms with an unidentified genus belonging to the family Ruminococcaceae, microorganisms belonging to the genus Anaerostipes, microorganisms belonging to the genus CAG-41, microorganisms belonging to the genus Barnesiella, microorganisms belonging to the genus Ruminococcus, microorganisms belonging to the genus Blautia, microorganisms belonging to the genus Eubacterium, microorganisms belonging to the genus Fusicatenibacter, microorganisms belonging to the genus Bacteroides, and microorganisms belonging to the genus Bifidobacterium. As for the "components effective for the proliferation of Faecalibacterium prausnitzii in the intestinal tract and the like," examples thereof include prebiotic components such as oligosaccharides and dietary fibers, as well as cocoa, barley, and Euglena gracilis.

In the food and/or beverage composition proposed in the present invention, it can also be provided and sold as a food and/or beverage with a health-related use displayed. The act of "displaying" includes all actions that inform the consumer of that use, and may involve expressions that directly recognize the use, or expressions that suggest or infer the use, and these expressions may be attached to the composition itself, or to the container, packaging material, or attached document containing the composition. Moreover, "display" may also refer to showing or advertising that the composition of the present invention is effective, as related information of the composition of the present invention, through flyers, pamphlets, point of purchase displays, catalogs, posters, books, DVDs, and other storage media, electronic bulletin boards, internet advertising, and the like.

<Methods for preventing deterioration in brain function, and the like>

The present invention also provides a method for preventing, treating, or improving deterioration in brain function in a subject, including administering to, or having the subject take, a composition (the aforementioned composition of the present invention) that contains as an active ingredient either Faecalibacterium prausnitzii according to the present invention, a component of the microorganism, a culture of the microorganism, or any combination thereof.

In the present invention, "brain function" means higher functions of the brain, encompassing functions (cognitive functions) achieved by internal brain information processes such as judgement, perception, computation, memory, understanding, learning, thinking, and language. " deterioration in brain function" can include, for example, mild cognitive impairment (MCI), decrease in learning and memory abilities, reduced judgement, diminished thinking ability, decreased perceptual and computational skills, language impairment, reduced concentration, or a decrease in productivity due to these factors (excluding depressive and anxiety states). Moreover, " deterioration in brain function" may also include central nervous diseases associated with it. Examples of such diseases include dementia, more specifically, Alzheimer's disease (Alzheimer's type dementia), Lewy body dementia, dementia in Parkinson's disease, and frontotemporal dementia; vascular dementia such as multi-infarct dementia, focal infarct dementia, and hereditary vascular dementia; early-onset dementia; and dementia caused by traumatic brain injury, substance or medicine use, HIV infection, prion disease, or Huntington's disease.

In the context of the present invention, "prevention" includes the suppression, delay, and inhibition of the recurrence of brain function deterioration. "Treatment" includes complete recovery from brain function deterioration. "Maintenance" includes the preservation of brain function, regardless of the presence of the disease. "Improvement" includes alleviating or enhancing symptoms related to brain function, inhibiting their progression, and suppressing their recurrence.

In the present invention, the "subject" for preventing deterioration in brain function, among other things, is animals, including humans. There are no specific restrictions for animals other than humans, and the subject may be various livestock, poultry, pets, experimental animals, and the like. Specific examples thereof include, but are not limited to, pigs, cattle, horses, sheep, goats, chickens, wild ducks, ostriches, domesticated ducks, dogs, cats, rabbits, hamsters, mice, rats, monkeys, among others. Furthermore, the subjects according to the present invention may be patients with diseases such as dementia, but do not necessarily need to be diagnosed with dementia or the like, and examples thereof include those who feel that their brain function has deteriorated, those who have or had a relative with dementia, the elderly, or those who wish to improve their memory and learning abilities. In addition, even those without any particular concerns about their brain function could consume it on a daily basis with the aim of preventing deterioration in brain function, improving brain function, or preventing dementia, among other purposes.

Also, the present technique can be used for therapeutic uses, or may be used for non-therapeutic uses. The concept of "non-therapeutic use" does not involve medical interventions, that is, treatments applied to the human body. For example, it includes health promotion, prevention of lifestyle diseases, and the like (such as potential risks in borderline areas).

When administering or taking a composition containing Faecalibacterium prausnitzii according to the present invention, a component of the microorganism, a culture of the microorganism, or any combination thereof as an active ingredient (the composition of the present invention described above), the dosage or intake amount is appropriately selected according to factors such as the subject's age, weight, symptoms of the aforementioned disorders, health conditions, type of composition (such as medicine or food and/or beverage), and the form of the active ingredient (for example, live bacteria, dead bacterial bodies, or bacterial body components).

The content or usage of the microorganism belonging to the genus Faecalibacterium in the present invention (live bacteria or dead bacterial bodies) is not particularly limited, but is in the range of $1 \times 10$ to the power of 2 to $1 \times 10$ to the power of 12, more preferably $1 \times 10$ to the power of 3 to $1 \times 10$ to the power of 11, and further preferably $1 \times 10$ to the power of 4 to $1 \times 10$ to the power of 10, in the composition. Note that the units are in CFU/g or CFU/mL, or cells (counts)/g or cells (counts)/mL. CFU stands for Colony forming unit.

In addition, the dosage per day of the microorganism belonging to the genus Faecalibacterium in the present invention (live bacteria or dead bacterial bodies) is not particularly limited, but is in the range of preferably $1 \times 10$ to the power of 3 to $1 \times 10$ to the power of 14, $1 \times 10$ to the power of 4 to $1 \times 10$ to the power of 13, further $1 \times 10$ to the power of 5 to $1 \times 10$ to the power of 13, and $1 \times 10$ to the power of 6 to $1 \times 10$ to the power of 12, per day. Note that the units are in CFU/day or cells (counts)/day.

In addition, the combination of upper limit value and lower limit value in the numerical range above is merely illustrative, and the combination thereof may be appropriately changed. Furthermore, when the active ingredient is a component of microorganism (bacterial body component), for instance, it can correspond to the amount of the bacterial body component relative to the amount of the bacterial body above.

Note that the dosage or intake amount of a composition containing the Faecalibacterium prausnitzii according to the present invention, a component of the microorganism, a culture of the microorganism, or any combination thereof as an active ingredient (the composition of the present invention described above) may be as described above, and this can be administered or taken once or several times in divided doses a day (for example, two or three times). Also, the duration of administration or intake can be discontinued depending on the degree of improvement in brain function, but from the viewpoint of preventing recurrence, it can also be continuously administered or taken without discontinuation. Note that as for "continuously," it can be continued daily or with intervals, but it is preferable to administer or take the Faecalibacterium prausnitzii or a composition containing the same as an active ingredient continuously on a daily basis for its effects.

<Composition and the like for inhibiting accumulation of amyloid-β>

As shown in the examples described later, the present invention can inhibit the accumulation of amyloid-β in the brain. Thus, the present invention also provides the following composition or method. Note that "amyloid β" and "form of amyloid-β to be accumulated" are as described above.

A composition for inhibiting the accumulation of amyloid-β in the brain of a subject, including as an active ingredient the Faecalibacterium prausnitzii according to the present invention, a component of the microorganism, or a culture of the microorganism.

A method for inhibiting the accumulation of amyloid-β in the brain of a subject, including administering to, or having the subject take, a composition (the aforementioned composition) that contains as an active ingredient either Faecalibacterium prausnitzii according to the present invention, a component of the microorganism, or a culture of the microorganism, or any combination thereof.

<Method for evaluating mild cognitive impairment or Alzheimer's disease>

As demonstrated in the examples described later, the results of a comparison of the gut microbiota between individuals with mild cognitive impairment or Alzheimer's disease patients and healthy individuals revealed gut microorganisms associated with the host's cognitive function. Therefore, the present invention also provides the following method for determining the onset of mild cognitive impairment or Alzheimer's disease using the amount of these gut microorganisms as an indicator.

<Method for evaluating mild cognitive impairment, 1>

(1) quantifying a microorganism in subject feces;
(2) comparing a value obtained by the quantification in step (1) with a corresponding value obtained by quantifying the microorganism in healthy subject feces; and
(3) determining that the subject is in a state of mild cognitive impairment if, as a result of the comparison in step (2), the quantitative value in the subject feces is lower than the corresponding value, wherein the microorganism is at least one microorganism selected from the group consisting of a microorganism belonging to the genus Faecalibacterium, a microorganism with an unidentified genus belonging to the family Ruminococcaceae, a microorganism belonging to the genus Anaerostipes, a microorganism belonging to the genus CAG-41, a microorganism belonging to the genus Barnesiella, and a microorganism belonging to the genus Ruminococcus.

<Method for evaluating mild cognitive impairment, 2>

(1) quantifying a microorganism in subject feces;
(2) comparing a value obtained by the quantification in step (1) with a corresponding value obtained by quantifying the microorganism in healthy subject feces; and
(3) determining that the subject is in a state of mild cognitive impairment if, as a result of the comparison in step (2), the quantitative value in the subject feces is higher than the corresponding value, wherein the microorganism is at least one microorganism selected from the group consisting of a microorganism belonging to the genus Prevotella.

<Method for evaluating Alzheimer's disease, 1>

(1) quantifying a microorganism in subject feces;
(2) comparing a value obtained by the quantification in step (1) with a corresponding value obtained by quantifying the microorganism in healthy subject feces; and
(3) determining that the subject is suffering from Alzheimer's disease if, as a result of the comparison in step (2), the quantitative value in the subject feces is lower than the corresponding value, wherein the microorganism is at least one microorganism selected from the group consisting of a microorganism belonging to the genus Blautia, a microorganism belonging to the genus Fusicatenibacter, a microorganism belonging to the genus Ruminococcus, and a microorganism belonging to the genus Eubacterium.

<Method for evaluating Alzheimer's disease, 2>

(1) quantifying a microorganism in subject feces;
(2) comparing a value obtained by the quantification in step (1) with a corresponding value obtained by quantifying the microorganism in healthy subject feces; and
(3) determining that the subject is suffering from Alzheimer's disease if, as a result of the comparison in step (2), the quantitative value in the subject feces is higher than the corresponding value, wherein the microorganism is at least one microorganism selected from the group consisting of a microorganism belonging to the genus Parabacteroides.

In the evaluation method of the present invention, examples of microorganisms belonging to the genus Faecalibacterium to be quantified include Faecalibacterium prausnitzii and "Faecalibacterium moorei."

Examples of microorganisms belonging to the genus Anaerostipes include Anaerostipes butyraticus, Anaerostipes caccae, Anaerostipes hadrus, and Anaerostipes rhamnosivorans.

Examples of microorganisms belonging to the genus Barnesiella include Barnesiella intestinihominis and Barnesiella viscericola.

Examples of microorganisms belonging to the genus Prevotella include Prevotella albensis, Prevotella amnii, Prevotella aurantiaca, Prevotella baroniae, Prevotella bergensis, Prevotella bivia, Prevotella brevis, Prevotella brunnea, Prevotella bryantii, Prevotella buccae, Prevotella buccalis, Prevotella cerevisiae, Prevotella colorans, Prevotella copri, Prevotella corporis, Prevotella dentalis, Prevotella dentasini, Prevotella denticola, Prevotella disiens, Prevotella enoeca, Prevotella falsenii, Prevotella fusca, Prevotella heparinolytica, Prevotella histicola, Prevotella hominis, Prevotella intermedia, Prevotella jejuni, "Prevotella koreensis," Prevotella loescheii, Prevotella maculosa, "Prevotella marseillensis," Prevotella marshii, "Candidatus Prevotella massiliensis," Prevotella melaninogenica, Prevotella micans, Prevotella multiformis, Prevotella multisaccharivorax, Prevotella nanceiensis, Prevotella nigrescens, Prevotella oralis, Prevotella oris, Prevotella oryzae, Prevotella oulorum, Prevotella pallens, Prevotella paludivivens, "Prevotella pectinovora," Prevotella phocaeensis, Prevotella pleuritidis, Prevotella rara, Prevotella ruminicola, Prevotella saccharolytica, Prevotella salivae, Prevotella scopes, Prevotella shahii, Prevotella stercorea, Prevotella timonensis, Prevotella veroralis, "Prevotella vespertina," Prevotella zoogleoformans, "Prevotella ihumii," "Prevotella lascolaii," and "Prevotella rectalis."

Examples of microorganisms belonging to the genus Blautia include Blautia argi, Blautia caecimuris, Blautia coccoides, Blautia faecicola, Blautia faecis, Blautia glucerasea, Blautia hansenii, Blautia hominis, Blautia hydrogenotrophica, Blautia luti, Blautia obeum, Blautia producta, Blautia schinkii, Blautia stercoris, Blautia wexlerae, "Blautia marasmi," "Blautia massiliensis," "Blautia phocaeensis," and "Blautia provencensis."

Examples of microorganisms belonging to the genus Fusicatenibacter include Fusicatenibacter saccharivorans.

Examples of microorganisms belonging to the genus Ruminococcus include Ruminococcus albus, Ruminococcus bromii, Ruminococcus callidus, Ruminococcus champanellensis, Ruminococcus flavefaciens, Ruminococcus gauvreauii, Ruminococcus gnavus, Ruminococcus lactaris, Ruminococcus torques, and "Ruminococcus bicirculans."

Examples of microorganisms belonging to the genus Eubacterium include Eubacterium aggregans, Eubacterium barkeri, Eubacterium brachy, Eubacterium callanderi, Eubacterium cellulosolvens, Eubacterium coprostanoligenes, Eubacterium infirmum, Eubacterium limosum, Eubacterium maltosivorans, Eubacterium minutum, Eubacterium multiforme, Eubacterium nodatum, Eubacterium oxidoreducens, Eubacterium plexicaudatum, Eubacterium pyruvativorans, Eubacterium ramulus, Eubacterium ruminantium, Eubacterium saphenum, Eubacterium siraeum, Eubacterium sulci, Eubacterium tenue, Eubacterium tortuosum, Eubacterium uniforme, Eubacterium ventriosum, Eubacterium xylanophilum, and Eubacterium yurii.

Examples of microorganisms belonging to the genus Parabacteroides include Parabacteroides acidifaciens, "Parabacteroides bouchesdurhonensis," Parabacteroides chartae, Parabacteroides chinchillae, Parabacteroides chongii, Parabacteroides distasonis, Parabacteroides faecis, Parabacteroides goldsteinii, Parabacteroides gordonii, Parabacteroides johnsonii, "Parabacteroides massiliensis," Parabacteroides merdae, "Parabacteroides pacaensis," "Parabacteroides provencensis," and "Parabacteroides timonensis."

Note that the names enclosed in "" indicates that they are "not validly published."

Examples of microorganisms with an unidentified genus belonging to the family Ruminococcaceae include microorganisms containing DNA having 80% or more homology to at least one of the nucleotide sequences set forth in SEQ ID NOs: 423 to 497.

Examples of microorganisms belonging to the genus CAG-41 include microorganisms containing DNA having 80% or more homology to at least one of the nucleotide sequences set forth in SEQ ID NOs: 498 to 504.

The term "homology" according to the present invention is as described above, and the homology of nucleotide sequences can be determined using alignment programs such as BLAST. For example, the homology of nucleotide sequences can refer to the homology between nucleotide sequences calculated using blastn, and more specifically to the homology between nucleotide sequences calculated using blastn with default parameters (that is, using the initially set parameters).

Note that the term "homology" here in relation to nucleotide sequences is, as described above, for example, 80% or more (more specifically, 81% or more, 82% or more, 83% or more, 84% or more), but preferably 85% or more (for example, 86% or more, 87% or more, 88% or more, 89% or more), more preferably 90% or more (for example, 91% or more, 92% or more, 93% or more, 94% or more), further preferably 95% or more (for example, 96% or more, 97% or more, 98% or more, 99% or more), and particularly preferably 100%.

In each evaluation method of the present invention, microorganisms to be quantified may be of at least one species, but preferably two or more species (for example, 3 or more species, 4 or more species), more preferably 5 or more species (for example, 6 or more species, 7 or more species, 8 or more species, 9 or more species), and further preferably 10 or more species.

There are no particular restrictions on the "subject" in the evaluation method of the present invention, which is the same as the "subject for preventing deterioration in brain function and the like" described above. Furthermore, there are no specific restrictions on the "healthy individuals" who serve as comparisons, but it is preferable if they share at least one characteristic, such as age, gender, race or nationality, or medical history excluding dementia, with the subjects.

"Quantification of microorganisms in feces" does not necessarily mean the amount (number) of the microorganisms themselves, and can be conducted, for example, by measuring the amount of substances specific to the microorganisms, reflecting the amount (number) of the microorganisms. Such substances may include oligonucleotides specific to the microorganisms (such as 16S rRNA genes and transcripts thereof), the constituent substances specific to the microorganisms (such as peptides, nucleic acids, sugars, lipids, and complexes thereof, which constitute the microorganisms), and the products generated by the microorganisms (such as secretions from the microorganisms and metabolites produced by the microorganisms).

When the substance is an oligonucleotide, it can be quantified using, for example, PCR (RT-PCR, real-time PCR, quantitative PCR), DNA microarray analysis methods, Northern blotting, next-generation sequencing methods (synthetic sequencing methods (sequencing-by-synthesis, for example, sequencing using Solexa Genome Analyzer or HiSeq (registered trademark) 2000 manufactured by Illumina), pyrosequencing methods (for example, sequencing using Sequencer GS FLX or FLX manufactured by Roche Diagnostics (454) (also known as 454 sequencing)), ligase reaction sequencing methods (for example, sequencing using SOLiD or 5500xl manufactured by Life Technologies), T-RFLP method, bead array method, in situ hybridization, dot blot, RNase protection assay method, mass spectrometry, genome PCR method, Southern blotting.

For other substances, for example, they can be quantified using methods that use antibodies (immunological methods), such as the ELISA method, immunoblotting, antibody array analysis, immunohistochemical staining, flow cytometry, imaging cytometry, radioimmunoassay, and immunoprecipitation.

Additionally, the values thus obtained by quantification can be not only absolute values but also relative values. Relative values may be, for example, quantity ratios based on the measurement method or measurement device used for detection (numerical values expressed in so-called arbitrary units (AU)). Also, relative values may be, for example, values representing the proportion of a microorganism within the entire gut microbiota thereof (relative abundance, occupancy rate), as demonstrated in the examples described later. Alternatively, values calculated using the amount of a reference gut microorganism as a standard can also be used. The "reference gut microorganism" according to the present invention is stably present in fecal samples and should be a microorganism whose amount shows little variation among different biological samples.

In addition, as demonstrated in the examples described later, the results of a comparison of a particular DNA amount between individuals with mild cognitive impairment and healthy individuals revealed DNAs associated with the host's cognitive function. Therefore, the present invention also provides the following method for determining the conditions of mild cognitive impairment using the amount of these DNAs as an indicator.

<Method for evaluating mild cognitive impairment, 3>

A method for evaluating mild cognitive impairment, comprising:

(1) quantifying DNA in subject feces;
(2) comparing a value obtained by the quantification in step (1) with a corresponding value obtained by quantifying the DNA in healthy subject feces; and
(3) determining that the subject is in a state of mild cognitive impairment if, as a result of the comparison in step (2), the quantitative value in the subject feces is lower than the corresponding value, wherein the DNA is at least one of the DNAs (a) to (i).

(a) a DNA including a nucleotide sequence encoding an amino acid sequence having 40% or more homology to an amino acid sequence encoded by a nucleotide sequence set forth in SEQ ID NO: 22 or 46,
(b) a DNA including a nucleotide sequence encoding an amino acid sequence having 40% or more homology to an amino acid sequence encoded by a nucleotide sequence set forth in SEQ ID NO: 158,
(c) a DNA including a nucleotide sequence encoding an amino acid sequence having 40% or more homology to an amino acid sequence encoded by a nucleotide sequence set forth in SEQ ID NO: 204,
(d) a DNA including a nucleotide sequence encoding an amino acid sequence having 40% or more homology to an amino acid sequence encoded by a nucleotide sequence set forth in SEQ ID NO: 205,

(e) a DNA including a nucleotide sequence encoding an amino acid sequence having 40% or more homology to an amino acid sequence encoded by a nucleotide sequence set forth in SEQ ID NO: 206,
(f) a DNA including a nucleotide sequence encoding an amino acid sequence having 40% or more homology to an amino acid sequence encoded by a nucleotide sequence set forth in SEQ ID NO: 365,
(g) a DNA including a nucleotide sequence encoding an amino acid sequence having 40% or more homology to an amino acid sequence encoded by a nucleotide sequence set forth in SEQ ID NO: 414,
(h) a DNA including a nucleotide sequence encoding an amino acid sequence having 40% or more homology to an amino acid sequence encoded by a nucleotide sequence set forth in SEQ ID NO: 420, and
(i) a DNA including a nucleotide sequence encoding an amino acid sequence having 40% or more homology to an amino acid sequence encoded by a nucleotide sequence set forth in SEQ ID NO: 421.

<Method for evaluating mild cognitive impairment, 4>

A method for evaluating mild cognitive impairment, comprising:

(1) quantifying DNA in subject feces;
(2) comparing a value obtained by the quantification in step (1) with a corresponding value obtained by quantifying the DNA in healthy subject feces; and
(3) determining that the subject is in a state of mild cognitive impairment if, as a result of the comparison in step (2), the quantitative value in the subject feces is higher than the corresponding value, and
the DNA is a DNA including a nucleotide sequence encoding an amino acid sequence having 40% or more homology to an amino acid sequence encoded by a nucleotide sequence set forth in SEQ ID NO: 297.

In each evaluation method of the present invention, the "DNA including a nucleotide sequence encoding an amino acid sequence having 40% or more homology to an amino acid sequence encoded by a nucleotide sequence set forth in SEQ ID NO: N" is as described above. In addition to this DNA, a DNA that contains a nucleotide sequence encoding an amino acid sequence, where one or more amino acids are substituted, deleted, added, and/or inserted, in the amino acid sequence encoded by the nucleotide sequence set forth in SEQ ID NO: N, or a DNA that hybridizes under stringent conditions with the DNA composed of the nucleotide sequence set forth in SEQ ID NO: N, can also be quantified, and the same applies to these DNAs as described above.
In each evaluation method of the present invention, DNAs to be quantified may be of at least one species, but preferably two or more species (for example, 3 or more species, 4 or more species), and more preferably 5 or more species (for example, 6 or more species, 7 or more species, 8 or more species, 9 or more species), in "Method for evaluating mild cognitive impairment, 3" above.
In the "Methods for evaluating mild cognitive impairment, 3 and 4" as well, the "subjects" are as described above. "Quantification of DNAs in feces" does not necessarily mean the amount (number) of the DNAs (genomic DNAs) themselves, and can be conducted, for example, by measuring the amount of substances reflecting the amount (number) of the DNAs. Such substances can include transcription products (mRNAs), cDNAs, and translation products (peptides). Moreover, as described above, those skilled in the art can quantify these substances by appropriately employing known quantification methods depending on the type of substance.
Additionally, the values thus obtained by quantification can be not only absolute values but also relative values. Relative values may be, for example, quantity ratios based on the measurement method or measurement device used for detection (numerical values expressed in so-called arbitrary units (AU)). Also, relative values may be, for example, values representing the proportion of a DNA to be quantified in the entire DNAs in the feces (relative abundance, occupancy rate). Alternatively, values calculated using the reference amount as a standard can also be used. The "reference amount" according to the present invention is stably present in fecal samples and should be a DNA whose amount shows little variation among different biological samples.
In the evaluation methods of the present invention ("Methods for evaluating Alzheimer's disease, 1 and 2" and "Methods for evaluating mild cognitive impairment, 1 and 2 to 4" described above), the obtained quantified values are compared with those (corresponding values) in healthy individuals. Such a comparison can be appropriately selected and performed by those skilled in the art using statistical analysis methods suitable for the above detection methods. Examples of statistical analysis methods include the t-test, Wilcoxon signed-rank test, analysis of variance (ANOVA), Kruskal-Wallis test, Mann-Whitney U test (Wilcoxon rank-sum test), odds ratios, hazard ratios, Fisher's exact tests, receiver operating characteristic analysis (ROC analysis), and classification and regression tree analysis (CART analysis). Furthermore, during the comparison, normalized or standardized and normalized data can also be used.
The determination of mild cognitive impairment or Alzheimer's disease is typically conducted by a physician (including those acting under a physician's instructions), and the aforementioned data regarding the amount of microorganisms, DNAs, or the like is useful for diagnosis, including helping the physician decide the timing of treatment and the like. Therefore, the methods of the present invention can be described as a method for collecting data on the aforementioned amount of microorganisms, DNAs, or the like for physician diagnosis, a method for presenting this data to the physician, a method for comparing and analyzing the aforementioned amount of microorganisms, amount of DNAs, or the like with the corresponding values, and a method for assisting the physician in diagnosing considering the patient's clinical symptoms and/or other test results.

[Examples]

Hereinafter, the present invention will be described in more detail based on the Examples, but the present invention is not limited to the following Examples.

(Example 1) Epidemiological Test

To select gut microorganisms (gut microbiota) involved in the brain function of the host, fecal samples were collected from individuals with normal brain functions such as cognitive functions (healthy individuals), individuals with mild cognitive impairment (MCI), and individuals suffering from Alzheimer's Disease (AD), and the composition of the gut microorganisms in each fecal sample was analyzed by sequencing the 16S rRNA sequence of these microorganisms.

Specifically, we invited 21 healthy individuals and 15 individuals with MCI from those who participated in a health check-up in 2015, in Kusatsu Town, Gunma Prefecture (Japan) to participate in our epidemiological study as subjects (all of whom were 65 years of age or older). In addition, we invited 7 individuals suffering from AD aged 65 or older to participate as subjects from among the residents of an old age home in Kusatsu Town, the users of Kusatsu Town's public care service, and those who attended the health check-ups that same year.

Note that from the subjects,

(1) those who had used antibiotics two weeks prior to the collection of feces,
(2) Those who had undergone surgery of the digestive tract in the past six months,
(3) those with a history of digestive tract disorders, such as inflammatory bowel disease, inflammatory bowel syndrome, tumors in the digestive tract, and gastrectomy,
(4) those with a history of diabetes, and
(5) those with a history of any significant neurological and psychiatric disorders other than AD

were excluded. Furthermore, all subjects were provided with informed consent in writing after receiving a detailed explanation of the research protocol approved by the ethics committee at Tokyo Metropolitan Geriatric Hospital and Institute of Gerontology.

The evaluation of the subjects was conducted by

(1) evaluating cognitive functions using the mini-mental state examination (MMSE), the Japanese version of the Montreal cognitive assessment (MoCA-J), and the clinical dementia rating (CDR),
(2) evaluating basic activities of daily living (ADL) using the Tokyo Metropolitan Institute of Gerontology Index of Competence (TMIG-IC),
(3) conducting medical interviews regarding the subjects' medical history and current medication, and
(4) using health check-up data, including blood tests and blood pressure.

The subjects were divided into three groups: a healthy individual group, an MCI group, and an AD group. Healthy individuals and individuals with MCI were diagnosed according to Petersen's criteria. Specifically, the subject were diagnosed as healthy if:(1) They did not complain of memory problems,

(2) MoCA-J $\geq$ 26,
(3) MMSE $\geq$ 24,
(4) the CDR score was 0,
(5) the ADL score was 5, or
(6) They had not been diagnosed with any neurological or psychiatric disorders.

Meanwhile, the subjects were diagnosed with MCI if:(1) They complained of memory problems,

(2) MoCA-J $\leq$ 25,
(3) MMSE $\geq$ 24,
(4) the CDR score was 0.5,

(5) the ADL score was 5, or

(6) They had not been diagnosed with any neurological or psychiatric disorders.

The affliction of AD was determined by physicians, based on the Diagnostic and Statistical Manual of Mental Disorders (DSM)-IV criteria, using medical interviews and questionnaires.

Feces were collected using a feces collection kit (manufactured by TechnoSuruga Laboratory Co., Ltd.) and then stored frozen. Then, in order to analyze the composition of gut microorganisms in each fecal sample using a next-generation sequencer, a library for that purpose was prepared. Specifically, DNA was first extracted from the collected feces. Using the obtained DNA as a template, the 16S rRNA region (V3-V4 region of 16S rRNA) was amplified for microorganism phylogenetic analysis by PCR. After PCR amplification, using the DNA fragment fractionation and collection kit for library preparation (manufactured by Thermo Fisher Scientific, product name: E-Gel (registered trademark) SizeSelect Gels), the amplified products containing the target region were extracted, indices were added, and then purified them using a DNA purification reagent kit (manufactured by Beckman Coulter, product name: Agencourt AMPure XP beads). Next, the concentration of the library solution thus prepared was measured, and quality control (QC) was conducted by electrophoresis using a Bioanalyzer (manufactured by Agilent Technologies). Then, the library solutions of each sample were mixed at equal molar ratios and sequencing was performed with a next-generation sequencer (MiSeq).

Based on the obtained sequencing results, bioinformatics analysis was conducted to calculate the proportion of gut microorganisms and the like. Specifically, the microbial community analysis program "QIIME 2" and the GTDB database were used to calculate phylogenetic composition, and significant taxa were extracted using R. Figs. 1 and 2 show the obtained results.

As a result of the above tests, 129 genera were obtained. Moreover, as shown in Figs. 1 and 2, it was possible to select 11 microorganisms that showing significant differences in group comparisons between healthy individuals vs MCI, and healthy individuals vs AD.

In particular, the relative abundance of microorganisms belonging to the genus Faecalibacterium, microorganisms with an unidentified genus belonging to the family Ruminococcaceae, microorganisms belonging to the genus Anaerostipes, microorganisms belonging to the genus CAG-41, microorganisms belonging to the genus Barnesiella, and microorganisms belonging to the genus Ruminococcus in the gut microbiota was found to be lower in individuals with MCI compared to healthy individuals (microorganisms located to the left of the line at 0 on the X-axis in Fig. 1, and above the line at P = 0.05). Meanwhile, the relative abundance of microorganisms belonging to the genus Prevotella was found to be higher in individuals with MCI compared to healthy individuals (microorganisms located to the right of the line at 0 on the X-axis in Fig. 1, and above the line at P = 0.05).

Furthermore, the microorganisms with an unidentified genus belonging to the family Ruminococcaceae refer to microorganisms that have been phylogenetically assigned to the family Ruminococcaceae in the aforementioned bioinformatics analysis but are unclassified with an unidentified genus, and specifically, these microorganisms have a DNA sequence set forth in any of SEQ ID NOs: 423 to 497, which encodes the V3-V4 region of 16S rRNA. Moreover, the microorganisms belonging to the genus CAG-41 have a DNA sequence set forth in any of SEQ ID NOs: 498 to 504, which encodes the V3-V4 region of the 16S rRNA region.

In addition, the relative abundance of microorganisms belonging to the genus Blautia, microorganisms belonging to the genus Fusicatenibacter, microorganisms belonging to the genus Ruminococcus, and microorganisms belonging to the genus Eubacterium in the gut microbiota was found to be lower in individuals with AD compared to healthy individuals (microorganisms located to the left of the line at 0 on the X-axis in Fig. 2, and above the line at P = 0.05). Meanwhile, the relative abundance of microorganisms belonging to the genus Parabacteroides was found to be higher in individuals with AD compared to healthy individuals (microorganisms to the right of the line at 0 on the X-axis in Fig. 2, and above the line at P = 0.05).

Therefore, it has been suggested that the above gut microorganisms are involved in brain functions (such as cognitive functions) of the host.

Next, to analyze the correlation between the relative abundance of microorganisms that showed significant differences in group comparisons of healthy individuals vs individuals with MCI, and healthy individuals vs individuals with AD, and the results of the cognitive function test MoCA-J, a Spearman's correlation analysis was conducted targeting healthy individuals and individuals with MCI. Table 1 shows the obtained results.

[Table 1]

| genus_name | spearman H_M MoCA cor | spearman_H_M_MoCA cor.p.val |
|---|---|---|
| k_Bacteria;p_Firmicutes_A;c_Clostridia;o_Oscillospirales; f_Ruminococcaceae;g_Faecalibacterium | 0.430690324 | 0.00980115 ∗ |

(continued)

| genus_name | spearman H_M MoCA cor | spearman_H_M_MoCA cor.p.val |
|---|---|---|
| k_Bacteria;p_Bacteroidota;c_Bacteroidia;o_Bacteroidales; f_Bacteroidaceae;g_Prevotella | -0.33724988 | 0.047567855 * |
| k_Bacteria;p_Firmicutes_A;c_Clostridia;o_Lachnospirales; f_Lachnospiraceae;g_Blautia_A | 0.351664576 | 0.03830188 * |
| k_Bacteria;p_Bacteroidota;c_Bacteroidia;o_Bacteroidales; f_Tannerellaceae;g_Parabacteroides | -0.07182876 | 0.681774995 |
| k_Bacieria;p_Firmicutes_A;c_Clostridia;o_Oscillospirales; f_Ruminococcaceae;_ | 0.419259814 | 0.012175863 * |
| k_Bacteria;p_Firmicutes_A;c_Clostridia;o_Lachnospirales; f_Lachnospiraceae;g_Anaerostipes | 0.430861615 | 0.009768799 * |
| k__Bacteria;p__Firmicutes_A;c__Clostridia;o__ Lachnospirales;f__Lachnospiraceae;g__Fusicatenibacter | 0.243427526 | 0.158795324 |
| k_Bacteria;p_Firmicutes_C;c_Negativicutes;o_ Selenomonadales;f_Selenomonadaceae;g_Megamonas | 0.421423108 | 0.011692342 * |
| k_Bacteria;p_Firmicutes_A;c_Clostridia;o_Oscillospirales; f_Ruminococcaceae;g_Ruminococcus_C | 0.343097534 | 0.043613226 * |
| k_Bacteria;p_Firmicutes;c_Bacilii;o_Lactobacillales;f_ Lactobacillaceae;g_Lactobacillus_B | 0.300580048 | 0.079344469 |
| k_Bacteria;p_Firmicutes_A;c_Clostridia;o_Monoglobales;f_ UBA1381;g_CAG-41 | 0.367303679 | 0.02996215 * |
| k_Bacteria;p_Firmicutes_A;c_Clostridia;o_Lachnospirales; f_Lachnospiraceae;g_Eubacterium_I | -0.10417383 | 0.551480994 |
| k_Bactena;p_Bacteroidota;c_Bacteroidia;o_Bacteroidales; f_Barnesiellaceae;g_Barnesiella | 0.436037778 | 0.00883308 * |
| k_Bacteria;p_Firmicutes_A;c_Clostridia;o_Lachnospirales; f_Cellulosilyticaceae;g_Niameybacter | -0.111212245 | 0.524761008 |
| k_Bacteria;p_Firmicutes_A;c_Clostridia;o_Oscillospirales; f_Oscillospiraceae;g_Papillibacter | -0.370202724 | 0.028593447 * |

As indicated in Table 1, the results of the analysis showed significant correlations in several genera. Particularly, Faecalibacterium prausnitzii (Fp) not only demonstrated a significant correlation but also had a high relative abundance, as shown in Fig. 1, significant differences were observed in the relative abundance between healthy individuals and individuals with MCI, so that in the following examples, the analysis focused on microorganisms belonging to the genus Faecalibacterium.

(Example 2) Isolation Test

As described above, in Example 1, gut microorganisms associated with brain function were found at the genus level. Therefore, the feces of healthy individuals and individuals with MCI were cultured under anaerobic conditions, and bacterial strains belonging to the above genus were isolated.

Specifically, first, the feces of healthy individuals and individuals with MCI were each diluted stepwise to $10^{-5}$ to $10^{-7}$ in an anaerobic chamber ($N_2$ = 90%, $CO_2$ = 5%, $H_2$ = 5%). The resulting fecal dilutions were each smeared on either YCFA agar medium, EG agar medium, or a medium with antibiotics added to these media. After culturing for 1 or 2 days at 37°C, approximately 7000 colonies from the grown colonies were picked up and, through several rounds of subculturing, the colonies were singled out. The singled-out isolated strains were preserved at -80°C in a 20% glycerol solution.

In addition, immediately before preserving the isolated strains, a portion of the bacterial bodies was suspended in a 5% Triton solution containing glass beads and homogenized, allowing for the extraction of the DNA of the isolated strains. Then, by performing PCR using a universal primer set (27F primer and 1492R primer) for amplifying the 16S rRNA gene, the 16S rRNA gene was amplified from the extracted DNA. Next, the sequencing of the PCR amplification products was conducted using the 27F primer. The obtained sequence data were subjected to BLAST analysis in three databases (NCBI, EzTaxon, Ltp), identifying the species of the isolated strains.

Specifically, 12 Faecalibacterium prausnitzii (Fp) strains (Fp1, Fp4, Fp14, Fp28, Fp40, Fp45, Fp77, Fp137, Fp360, Fp944, Fp1043, Fp1160) were isolated and identified. Only Fp944 was derived from MCI, and the remaining 11 strains were derived from healthy individuals.

(Example 3) Efficacy Animal Test 1

Regarding the 12 Fp strains among the gut microorganisms related to brain function, which were isolated and identified in Example 2, these bacterial strains were administered for a short period (11 days) to mice administered with amyloid-$\beta$ into their cerebral ventricles, and the degree of improvement in learning and memory disorders in these mice was evaluated using the Y-maze test and the passive avoidance test.

Specifically, the Fp strains were revived in a YCFA medium within an anaerobic chamber, and cultured at 37°C for 24 hours. Then, the culture fluid was subcultured to LYBHI medium (inoculum volume: 5%) and further cultured for another 24 or 48 hours. The resulting culture fluid was then concentrated by centrifugation and used as the test substance. Note that the test substance was adjusted to a turbidity of 0.6 (McFarland Scale) at a 20-fold dilution (equivalent to 6.6 $\times$ 10 to the power of 8 cells/mouse). The test substance was stored at -80°C until administered to the mice.

Mice, specifically Slc: ddY male mice (5 weeks old), were used. Test substances such as the Fp strains were orally administered repeatedly, once a day for 11 days. On day 3 of administration, amyloid-$\beta$ was singly administered into the cerebral ventricles of the brain. Additionally, as controls, the following groups were established.

Control Group 1: a sham surgery group where water for injection was administered into the cerebral ventricles, and a medium (physiological saline) was administered orally,

Control Group 2: a medium control group where amyloid-$\beta$ was administered into the cerebral ventricles, and a medium (physiological saline) was administered orally, Control Group 3: a medium control group where amyloid-$\beta$ was administered into the cerebral ventricles, and a medium (culture medium) was administered orally, and Control Group 4: a control group where amyloid-$\beta$ was administered into the cerebral ventricles, and Aricept (donepezil hydrochloride) was administered orally at 0.5 mg/kg body weight.

Note that each test substance group and the aforementioned control groups were set up with 9 mice per group.

A Y-maze test was conducted 1 hour after administration of the test substances on day 9 of administration, and a passive avoidance test was conducted 1 hour after administration of test substances on days 10 and 11 of administration.

In the Y-maze test, a plastic Y-shaped maze was used, which had three arms each 39.5 cm in length, a floor 4.5 cm in width, and a wall 12 cm in height, with each arm branching off at 120 degrees. The mice were placed in one of the arms of the Y-maze and allowed to explore freely within the maze for seven minutes. The order of arms entered by the mice within the measurement time was recorded, and the number of entries into an arm was counted to get the total number of entries. Subsequently, the number of times the mice entered different arms consecutively three times was counted, and this number was called the spontaneous alternation behavior count. Furthermore, the spontaneous alternation behavior rate was calculated using the formula below. Measurements were conducted in a (blind) situation, with the observer unaware of the group information.

$$\text{Spontaneous alternation behavior rate (\%)} = [\text{Spontaneous alternation behavior count} / (\text{Total number of entries} - 2)] \times 100$$

Fig. 3 shows the obtained results.

In the passive avoidance test, a step-through type passive avoidance reaction device was used, equipped with a bright room (W: 100 $\times$ D: 100 $\times$ H: 300 mm) separated by a central guillotine door, and a dark room (W: 240 $\times$ D: 245 $\times$ H: 300 mm) that delivers electrical stimulation from the floor grid. The animal was placed in the bright room, and 10 seconds later, the guillotine door was quietly opened, and the time until the animal entered the dark room (reaction latency time) was measured. The acquisition trial (10 days post-administration) involved closing the guillotine door and delivering electrical stimulation (0.2 mA, 2 sec, scramble method) as soon as the animal entered the dark room, and confirming the presence or absence of the animal's cry during the electric stimulus load. The measurement of reaction latency time was limited to a maximum of 300 seconds. The retention trial (11 days post-administration) ended when the animal entered the dark room or after 300 seconds had passed in the bright room. Fig. 4 shows the obtained results.

As a result of the aforementioned Y-maze test, as shown in Fig. 3, significant statistical improvements in spontaneous alternation behavior rate were observed in Fp14, Fp28, Fp77, and Fp360, compared to the culture administration group. In addition, from the results of the passive avoidance test, as shown in Fig. 4, a significant statistical increase in reaction latency time was observed in Fp360, compared to the culture administration group. Furthermore, following Fp360, the possibility of an increase in reaction latency time was also observed in Fp1043 and Fp14. Therefore, these Fp strains, particularly Fp14 and Fp360, which were suggested to be effective in both the Y-maze test and the passive avoidance test, were found to potentially have an improvement effect on the deterioration in brain function, such as learning and memory disorders.

(Example 4) Efficacy Animal Test 2

Regarding Fp14 and Fp360, which were suggested to have an improvement effect on the deterioration of brain function in Example 3, culture supernatants and dead bacterial bodies thereof were also evaluated for the degree of improvement in learning and memory disorders. The culture supernatants and dead bacterial bodies were prepared by the following method.

<Method for preparing culture supernatants>

The Fp strain stock bacterial solutions were cultured in a YCFA medium within an anaerobic chamber (37°C, 24 hours). Furthermore, the culture fluid was subcultured in LYBHI medium (37°C, 24 hours) so that the inoculum volume was 5%. The culture fluid was centrifuged ($800 \times g$, room temperature, 10 minutes) and filtered (0.22 um diameter) to obtain a culture supernatant. The culture supernatants were stored at -80°C until administered to the animals.

<Method for preparing dead bacterial body solutions>

The Fp strain stock bacterial solutions were cultured in a YCFA medium within an anaerobic chamber (37°C, 24 hours). Furthermore, the culture fluid was subcultured in LYBHI medium (37°C, 24 hours) so that the inoculum volume was 5%. The culture fluid was centrifuged ($800 \times g$, room temperature, 10 minutes) to remove the supernatant, followed by two rounds of centrifugal washing using physiological saline. The bacterial bodies were suspended so as to match the turbidity in the preparation of live bacteria samples, using physiological saline, which was subjected to heat treatment (70°C, 30 minutes) to obtain a solution of dead bacterial bodies. The solution of dead bacterial bodies was stored at -80°C until administered to the animals.

Then, the culture supernatants and solution of dead bacterial bodies thus prepared were, as test substances, administered for a short period (11 days) to mice administered with amyloid-β into their cerebral ventricles, in a manner similar to that described in Example 3, and the degree of improvement in learning and memory disorders in these mice was evaluated using a Y-maze test and a passive avoidance test (each test substance group and each control group were set with 12 mice per group). Figs. 5 and 6 show the obtained results.

Based on the results of the Y-maze test, as shown in Fig. 5, an improvement effect and improvement trend was observed for both Fp14 and Fp360, as also demonstrated in Example 3 (Fig. 3), through the administration of live bacteria. Furthermore, by administering dead bacterial bodies of Fp360, an improvement trend in the rate of spontaneous alternation behavior was observed compared to the culture administration group.

In addition, based on the results of the passive avoidance test, as shown in Fig. 6, an improvement effect was observed for Fp360, as also demonstrated in Example 3 (Fig. 4), through the administration of live bacteria. Meanwhile, regarding Fp14, it was revealed that the reaction latency time was statistically significantly increased by using dead bacterial bodies as compared with the physiological saline-administered group.

(Example 5) Whole Genome Analysis 1

In Examples 3 and 4, an attempt was made to compare the entire genome along the pipeline shown in Fig. 7, between the Fp strains (Fp14 and Fp360) that suggested effectiveness against brain function deterioration in both the Y-maze test and passive avoidance test, and the Fp strains (the 10 strains other than the aforementioned 2 strains) that were not recognized in both of the tests, in order to extract genes characteristic of the former.

Specifically, first, the Fp strains were revived in a YCFA medium within an anaerobic chamber, and cultured at 37°C for 24 hours. Then, after centrifugation at $800 \times g$ for 10 minutes at 4°C, the supernatant was discarded and washed with anaerobic PBS. After that, centrifugation and PBS washing were repeated twice, and the bacterial body pellets were cryopreserved.

Genomic DNA was prepared from the bacterial body pellets using an anion exchange column for genome DNA purification and its dedicated buffer (trade name: NucleoBond (registered trademark) AXG 20, NucleoBond Buffer Set III, both manufactured by Macherey-Nagel). Furthermore, the genomic DNA was physically fragmented using a DNA fragmentation tube (trade name: g-TUBE, manufactured by Covaris).

To subject these fragmented DNAs to next-generation sequencing (PacBio sequencing), both ends of the fragmented DNA were first smoothed, and then SMRTbell adaptors were connected to prepare a sequencing library as a sequence template (SMRTbell Template). The prepared SMRTbell Template was size-distributed by pulse field electrophoresis, and size selection was conducted using an automatic DNA fragment gel extraction device (trade name: BluePippin, manufactured by Sage Science) or DNA purification reagent (trade name: AMPure XP, manufactured by Beckman Coulter). Next, a sequencing primer with a complementary sequence to the SMRTbell adapters at both ends of the sequencing library, and a DNA polymerase were combined to create a sequence template. This template was coupled with magnetic beads and applied to SMRT (registered trademark) Cells, where the sequence template was fixed within the wells (Zero-mode waveguides; ZMW), and sequencing reactions were then performed by a single molecule real-time DNA sequencer (trade name: PacBio RS II, manufactured by Pacific Biosciences).

Then, the reads obtained in the sequence were assembled de novo using the attached software of the sequencer, SMRT Analysis. For the contigs obtained through assembly, the following processes were conducted using the Circulator pipeline.

Firstly, those whose full length was included in other contigs were removed. Then, for those whose both ends overlapped, circularization was performed, followed by correction of the genome sequence start position using the dnaA gene sequence. After that, re-polishing was carried out using Quiver. In the re-polishing, mapping of subreads was performed against the contigs (consensus sequences) after circularization and other processes, and the consensus sequences were corrected based on these results. Furthermore, the completeness of the genome and contamination were judged by CheckM (v1.0.08).

Then, the prediction of genes (coding regions (CDS)) in the obtained circular whole genome sequence and the annotation of the genome were carried out using Prokka (v1.12).

Next, based on the predicted gene information, orthologs were identified using two different ortholog search tools, Roary (v3.12.0) and Orthofinder (v2.3.11). Note that Roary and Orthofinder were conducted under the following conditions.

(1) Roary

Orthologs were primarily clustered at an identity of 70% or more in BLASTP homology (see Bioinformatics, volume 31, issue 22, issued on November 15, 2015, pp. 3691 to 3693).

(2) Orthofinder

Orthologs were clustered at a unique threshold of Orthofinder. More specifically, after calculating Orthofinder's unique score (normalized score) using the bit score of all vs all blastp, they were clustered as the same orthologs if they were RBNH (Reciprocal Best length-Normalized hit = the normalized score is the best hit in both directions) in a certain query, or if the normalized score was equal to or above the lowest RBNH of a certain query (see Genome Biol. August 6, 2015; 16(1): 157).

In addition, the aforementioned predicted genes were annotated to KEGG genes and KEGG orthologs (KO) using Diamond (v0.9.10.111, identity threshold > 40, score > 70, and coverage > 80) (the referenced KO data is as of May 2017). Subsequently, the KOs were assigned to orthologs identified by Roary or Orthofinder.

Additionally, based on the predicted gene information, the prediction of protein domains was performed using InterProScan (v5.40-77.0) and TMHMM (v2.0). Note that in TMHMM, if the entire sequence was classified as inside or outside, it was predicted to be an intracellular protein or an extracellular protein without including a cell membrane protein. Meanwhile, if the entire sequence was classified as having at least one transmembrane helix, it was predicted to be a transmembrane protein.

Then, in this way, orthologs of sequences (genes) unique to each Fp strain and orthologs containing KO unique to each Fp strain were identified (hereinafter collectively referred to as "unique orthologs").

Table 2 shows the extracted orthologs of the sequences unique to both Fp14 and Fp360 Fp strains.

[Table 2]

| Analysis Software | Fp14 | SEQ ID NO: | Fp360 | SEQ ID NO: |
|---|---|---|---|---|
| Extracted with Roary 70 alone | ICBIDGAJ_00324 | 9 | BLCPAMOF_01513 | 30 |
| | ICBIDGAJ_00393 | 19 | BLCPAMOF_00265 | 33 |
| | ICBIDGAJ_00394 | 17 | BLCPAMOF_00266 | 34 |
| | ICBIDGAJ_00623 | 3 | BLCPAMOF_00817 | 43 |
| | ICBIDGAJ_01076 | 6 | BLCPAMOF_00918 | 53 |
| | ICBIDGAJ_01077 | 7 | BLCPAMOF_00907 | 35 |
| | ICBIDGAJ_01078 | 2 | BLCPAMOF_00908 | 39 |
| | ICBIDGAJ_01749 | 25 | BLCPAMOF_01424 | 41 |
| | ICBIDGAJ_01867 | 23 | BLCPAMOF_00910 | 50 |
| | ICBIDGAJ_02197 | 24 | BLCPAMOF_01019 | 45 |
| | ICBIDGAJ_02198 | 26 | BLCPAMOF_01020 | 48 |
| | ICBIDGAJ_02223 | 8 | BLCPAMOF_00484 | 40 |
| | ICBIDGAJ_02224 | 4 | BLCPAMOF_00483 | 37 |
| | ICBIDGAJ_02225 | 13 | BLCPAMOF_00482 | 38 |
| | ICBIDGAJ_02239 | 16 | BLCPAMOF_02131 | 56 |
| | ICBIDGAJ_02240 | 14 | BLCPAMOF_02130 | 57 |
| | ICBIDGAJ_02241 | 27 | BLCPAMOF_02129 | 36 |
| | ICBIDGAJ_02242 | 12 | BLCPAMOF_02128 | 42 |
| Extracted with both Roary 70 and Orthofinder | ICBIDGAJ_02236 | 22 | BLCPAMOF_02134 | 46 |
| | ICBIDGAJ_02237 | 21 | BLCPAMOF_02133 | 54 |
| | ICBIDGAJ_02238 | 18 | BLCPAMOF_02132 | 55 |
| Extracted with orthofinder alone | ICBIDGAJ_00328 | 15 | BLCPAMOF_02753 | 31 |
| | | | BLCPAMOF_02754 | 49 |
| | ICBIDGAJ_01075 | 5 | BLCPAMOF_00916 | 52 |
| | | | BLCPAMOF_00917 | 51 |
| | ICBIDGAJ_01337 | 20 | BLCPAMOF_00378 | 44 |
| | ICBIDGAJ_01576 | 1 | BLCPAMOF_00329 | 32 |
| | ICBIDGAJ_01754 | 11 | BLCPAMOF_01430 | 29 |
| | ICBIDGAJ_01852 | 10 | BLCPAMOF_01009 | 47 |

Furthermore, "ICBIDGAJ" and "BLCPAMOF" indicate IDs given to each gene predicted by Prokka in the whole genome analysis of Fp14 and Fp360. The sequences of these genes are set forth in SEQ ID NOs: 1 to 27 and 29 to 57, respectively. Also, in Table 2, genes placed side by side (for example, ICBIDGAJ_00324 (SEQ ID NO: 9) and BLCPAMOF_01513 (SEQ ID NO: 30)) are in an orthologous relationship.

Additionally, ICBIDGAJ_00537 and BLCPAMOF_00623 (SEQ ID NOs: 28 and 422) were extracted as genes corresponding to the unique KO1684 that characterizes Fp14 and Fp360.

Based on the results above, 28 unique orthologs characterizing Fp14 and Fp360 were extracted. Among them, the three orthologs commonly extracted in both analyses using Roary and Orthofinder were found to be contiguous on the genome, as shown in Fig. 8. Furthermore, as a result of the domain analysis, these three orthologs were predicted to encode transmembrane domains and extracellular domains according to TMHMM.

(Example 6) Whole Genome Analysis 2

Next, following the same method as described in Example 5, a comparison of the entire genome was made between Fp14 and 11 other strains, including Fp360, and genes and KOs characteristic only of Fp14 were extracted.

As a result, in both Roary and Orthofinder analyses, 150 orthologs unique to Fp14, corresponding to the DNA sequence set forth in SEQ ID NOs: 58 to 207, were successfully selected. Among them, as a result of focusing on the consecutive genes within the unique 146 genes characterizing Fp14 only (set forth in SEQ ID NOs: 58 to 203), areas were recognized, where genes encoding unknown extracellular proteins and filamentous-related proteins were lined up in sequence (see Fig. 9).

In addition, as unique KOs characterizing only Fp14, 8 KOs corresponding to the nucleotide sequences set forth in SEQ ID NOs; 60, 110, 140, and 204 to 207 were extracted. Among these, KO1226 (ICBIDGAJ_00755 (SEQ ID NO: 205)), KO2818, and KO2819 (ICBIDGAJ_00754 (SEQ ID NO: 204)), along with KO3486 (ICBIDGAJ_00753 (SEQ ID NO: 206)), were KOs related to trehalose, and as shown in Fig. 10, they were adjacent to unique genes characterizing only Fp14 (ICBIDGAJ_00757 and 00756). Also, KO2819 and KO1226 are KOs involved in a metabolic pathway that absorbs extracellular trehalose and produces trehalose-6P and the like (pathway which, starting from extracellular trehalose, via trehalose-6P, generates D-glucose-6P).

(Example 7) Whole Genome Analysis 3

Next, following the same method as described in Example 5, a comparison of the entire genome was made between Fp360 and 11 other strains, including Fp14, and genes and KOs characteristic only of Fp360 were extracted.
As a result, in both Roary and Orthofinder analyses, 214 orthologs unique to Fp360, corresponding to the DNA sequence set forth in SEQ ID NOs: 208 to 421, were successfully selected. Among them, 197 unique genes were extracted that characterize only Fp360 (set forth in SEQ ID NOs: 208 to 404). In addition, as unique KOs characterizing only Fp360, 36 KOs corresponding to the nucleotide sequences set forth in SEQ ID NOs; 212, 228, 229, 238, 247, 249, 257, 274, 276, 279, 290, 296, 309, 335, 336, 337, 342, 371, 377, and 405 to 421, respectively, were extracted. In these, KOs associated with cell division and the like were observed as KOs arranged continuously.

(Example 8) Metagenomic Analysis

The relative abundance was detected of orthologs unique to the Fp strain (Fp14 and/or Fp360) extracted in Examples 5 to 7, which was confirmed to be effective against brain function deterioration, in the gut microbiota of the gut metagenome samples prepared in the epidemiological test of Example 1. Then these relative abundances were compared between groups (comparisons between healthy individuals and individuals with MCI, and between healthy individuals and AD individuals).
Specifically, total metagenomic shotgun sequencing was first performed. The sequencing library was prepared using the TruSeq Nano DNA library preparation kit (manufactured by Illumina). The quality of the library was confirmed using Agilent 2100 Bioanalyzer. The total metagenomic shotgun sequencing targeting the fecal samples obtained in Example 1 was performed on HiSeq2500 Platform (manufactured by Illumina). For all samples, paired-end sequencing for a target data size of 15.0 Gb was conducted at a read length of 150 bp.
As a result, a total of 6,421,909,374 (average 149,346,730) paired-end reads covered a total of 963,286,406,100 (average 22,402,009,444) base pairs.
Next, for quality control, raw reads containing the character "N" (unidentified base pairs) were discarded. Reads containing bacteriophage phiX DNA sequences were identified and discarded by mapping the reads using Bowtie 2 (version 2.2.9) with its option (fast-local). Subsequently, Cutadapt (version 1.9.1) was used to trim the reads of adapter sequences and primer sequences. Reads containing a quality value of 17 or less in sequence were trimmed at the 3'-end by the Cutadapt program. Then, reads shorter than 50 base pairs were discarded, and reads with an average quality value of 25 or less were discarded. Next, the reads were mapped to the human genome (gi24 numbers: from 568336000 to 568336023, http://www.ncbi.nlm.nih.gov/nuccore/568336023/, GRCh38) using Bowtie 2 (version 2.2.9). Then, the mapped reads were considered to be derived from the human genome and were discarded. Finally, unpaired reads were discarded.
As a result, paired-end reads totaling 6,229,896,106 (average 144,881,305) with 934,484,415,900 (average 21,732,195,719) base pairs were obtained as high-quality reads and used for the analysis of relative abundance in the following gut microbiota.
The relative abundance of unique orthologs in the gut microbiota was evaluated based on the TPM (transcripts per million) values calculated by Kallisto (v0.46.2). Then, the relative abundance of these unique orthologs was compared between groups (healthy individuals and individuals with MCI, as well as between healthy individuals and AD individuals) using R (3.6.3).
As a result, as shown in Fig. 8, for example, the relative abundance of the orthologs of the sequences ICBIDGAJ_02236 and BLCMPAMOF_02134, which were unique genes characterizing Fp14 and Fp360 and were predicted to be transmembrane proteins, was significantly higher in healthy individuals than in individuals with MCI in the gut microbiota.
As shown in Fig. 10, the relative abundance of the ortholog of ICBIDGAJ_00756, which was unique to Fp14 and was predicted to be an extracellular protein, was significantly higher in healthy individuals than in individuals with MCI in the gut microbiota. Furthermore, the relative abundance of this ortholog tended to be significantly higher in healthy individuals compared to AD patients.

As shown in Fig. 10, the relative abundance in the gut microbiota of orthologs that fell (assigned) to Fp14-unique KOs and to which ICBIDGAJ_00753, 00754, and 00755 belonged was significantly higher in healthy individuals than in individuals with MCI.

The relative abundance of the ortholog unique to Fp360, to which BLCPAMOF_02713 belonged, was significantly higher in healthy individuals than in individuals with MCI in the gut microbiota.

The relative abundance of the ortholog, which was unique to Fp360, fell to cell division-related KO, and to which BLCPAMOF_00796 estimated to be a transmembrane protein belonged, was significantly higher in healthy individuals than in individuals with MCI in the gut microbiota.

The relative abundance of the ortholog, which was unique to Fp360, fell to cell division-related KO, and to which BLCPAMOF_01742 predicted to be an intracellular protein belonged, was significantly higher in healthy individuals than in individuals with MCI in the gut microbiota.

The relative abundance of the ortholog unique to Fp360, to which BLCPAMOF_02824 belonged, was significantly higher in healthy individuals than in individuals with MCI in the gut microbiota, in the analysis by Orthofinder alone.

(Example 5) Long-term Animal Efficacy Test

The above Fp14 culture fluid was administered to AD model mice for a long period of time (13 months), and the amount of amyloid-β in brain tissue was measured by immunohistochemical staining analysis.

Specifically, the Fp14 was revived in a YCFA medium within an anaerobic chamber, and cultured at 37°C for 24 hours. Then, the culture fluid was subcultured to LYBHI medium (inoculum volume: 5%) and further cultured for another 24 or 48 hours. The resulting culture fluid was then concentrated by centrifugation and used as the test substance. The test substance was stored at -80°C until administered to the mice.

As AD model mice, APP-tg mice (8 weeks old, male, SPF grade) were used. These mice were orally administered with the test substance 4 times a week for 13 months. In each oral administration, 357 pl (a bacterial amount with a turbidity (McFARLAND SCALE) at a 20-fold dilution of 0.6, equivalent to $6.6 \times 10$ to the power of 8 cells/mouse) was administered. After completing the mouse breeding, the mouse brain was sampled. The sampled brain was split in the median sagittal plane and one hemisphere was frozen and stored at -80°C. It was then immersed and fixed in PFA fixative (0.1% glutaraldehyde + 4% paraformaldehyde).

After fixing the hemisphere with PFA, it was embedded following sucrose substitution, and 40 um thin sections were prepared by freeze-cutting using a cryostat (manufactured by Leica, CM1860). After washing the floating sections twice in PBS, they were autoclaved at 121°C for 15 minutes in citrate buffer to perform antigen activation. After confirming that the citrate buffer had returned to room temperature, the samples were washed once with PBS, and membrane permeability treatment was performed by reacting with 0.1% TritonX-100 (w/v) added PBS (hereafter T-PBS) for 30 minutes. Blocking was conducted using Blocking One Histo (manufactured by Nacalai Tesque, Inc.), and the samples were reacted overnight at 4°C with a biotin-labeled antibody (Anti-β-Amyloid (17-24), Mouse-Mono (4G8), Biotin (manufactured by Tomy Digital Biology Co., Ltd., model number: 800704)). The target protein was labeled by reacting with fluorescently labeled streptavidin, and fluorescent images were obtained using a confocal laser microscope (manufactured by Zeiss, LSM700). Based on the obtained image data, the senile plaque occupancy rate, which reflects the amount of amyloid-β accumulated in the cortex and hippocampus, was analyzed.

As shown in Fig. 11, the administration of Fp14 was observed to tend to decrease the amount of amyloid-β accumulated in the hippocampus of the AD model mice. The onset and progression of AD are believed to be due to the fibrillization, aggregation, and accumulation of amyloid-β produced in the brain. Therefore, in conjunction with the results of Example 3 (Efficacy Animal Test), Fp14 at least has demonstrated potential effectiveness for the prevention, treatment, and improvement of AD.

[Industrial Applicability]

As described above, according to the present invention, it becomes possible to diagnose dementia or mild cognitive impairment, or precursor stages thereof, using the amount of specific gut microorganisms as an indicator. In particular, in the present invention, it also becomes possible to discern and evaluate the decline in cognitive function, as the gut microorganisms associated with mild cognitive impairment and Alzheimer's disease have been clarified.

In addition, according to the present invention, by administering a Faecalibacterium prausnitzii strain having a specific DNA sequence, it becomes possible to prevent or treat a deterioration in brain function, particularly a decline in cognitive function, in a subject. Furthermore, it also becomes possible to maintain or improve brain function, especially cognitive function.

Therefore, the present invention is useful in the development of diagnostic methods, preventive methods, and therapeutic methods, and the like, for deterioration in brain function, particularly dementia.

[Accession Number]

(1) Identification: Fp_0014
(2) Accession number: NITE BP-03169
(3) Accession date: March 6, 2020
(4) Depositary organization: NITE Patent Microorganisms Depositary, National Institute of Technology and Evaluation
(1) Identification: Fp_0360
(2) Accession number: NITE BP-03196
(3) Accession date: April 7, 2020
(4) Depositary organization: NITE Patent Microorganisms Depositary, National Institute of Technology and Evaluation

**Claims**

1. A composition for preventing or treating deterioration in brain function, or for maintaining or improving brain function, including Faecalibacterium prausnitzii, a component of the microorganism, or a culture of the microorganism, having at least one of the following DNAs (1) to (28), as an active ingredient

(1) a DNA including a nucleotide sequence encoding an amino acid sequence having 40% or more homology to an amino acid sequence encoded by a nucleotide sequence set forth in SEQ ID NO: 22 or 46,
(2) a DNA including a nucleotide sequence encoding an amino acid sequence having 40% or more homology to an amino acid sequence encoded by a nucleotide sequence set forth in SEQ ID NO: 21 or 54,
(3) a DNA including a nucleotide sequence encoding an amino acid sequence having 40% or more homology to an amino acid sequence encoded by a nucleotide sequence set forth in SEQ ID NO: 18 or 55,
(4) a DNA including a nucleotide sequence encoding an amino acid sequence having 40% or more homology to an amino acid sequence encoded by a nucleotide sequence set forth in SEQ ID NO: 9 or 30,
(5) a DNA including a nucleotide sequence encoding an amino acid sequence having 40% or more homology to an amino acid sequence encoded by a nucleotide sequence set forth in SEQ ID NO: 19 or 33,
(6) a DNA including a nucleotide sequence encoding an amino acid sequence having 40% or more homology to an amino acid sequence encoded by a nucleotide sequence set forth in SEQ ID NO: 17 or 34,
(7) a DNA including a nucleotide sequence encoding an amino acid sequence having 40% or more homology to an amino acid sequence encoded by a nucleotide sequence set forth in SEQ ID NO: 3 or 43,
(8) a DNA including a nucleotide sequence encoding an amino acid sequence having 40% or more homology to an amino acid sequence encoded by a nucleotide sequence set forth in SEQ ID NO: 6 or 53,
(9) a DNA including a nucleotide sequence encoding an amino acid sequence having 40% or more homology to an amino acid sequence encoded by a nucleotide sequence set forth in SEQ ID NO: 7 or 35,
(10) a DNA including a nucleotide sequence encoding an amino acid sequence having 40% or more homology to an amino acid sequence encoded by a nucleotide sequence set forth in SEQ ID NO: 2 or 39,
(11) a DNA including a nucleotide sequence encoding an amino acid sequence having 40% or more homology to an amino acid sequence encoded by a nucleotide sequence set forth in SEQ ID NO: 25 or 41,
(12) a DNA including a nucleotide sequence encoding an amino acid sequence having 40% or more homology to an amino acid sequence encoded by a nucleotide sequence set forth in SEQ ID NO: 23 or 50,
(13) a DNA including a nucleotide sequence encoding an amino acid sequence having 40% or more homology to an amino acid sequence encoded by a nucleotide sequence set forth in SEQ ID NO: 24 or 45,
(14) a DNA including a nucleotide sequence encoding an amino acid sequence having 40% or more homology to an amino acid sequence encoded by a nucleotide sequence set forth in SEQ ID NO: 26 or 48,
(15) a DNA including a nucleotide sequence encoding an amino acid sequence having 40% or more homology to an amino acid sequence encoded by a nucleotide sequence set forth in SEQ ID NO: 8 or 40,
(16) a DNA including a nucleotide sequence encoding an amino acid sequence having 40% or more homology to an amino acid sequence encoded by a nucleotide sequence set forth in SEQ ID NO: 4 or 37,
(17) a DNA including a nucleotide sequence encoding an amino acid sequence having 40% or more homology to an amino acid sequence encoded by a nucleotide sequence set forth in SEQ ID NO: 13 or 38,

(18) a DNA including a nucleotide sequence encoding an amino acid sequence having 40% or more homology to an amino acid sequence encoded by a nucleotide sequence set forth in SEQ ID NO: 16 or 56,

(19) a DNA including a nucleotide sequence encoding an amino acid sequence having 40% or more homology to an amino acid sequence encoded by a nucleotide sequence set forth in SEQ ID NO: 14 or 57,

(20) a DNA including a nucleotide sequence encoding an amino acid sequence having 40% or more homology to an amino acid sequence encoded by a nucleotide sequence set forth in SEQ ID NO: 27 or 36,

(21) a DNA including a nucleotide sequence encoding an amino acid sequence having 40% or more homology to an amino acid sequence encoded by a nucleotide sequence set forth in SEQ ID NO: 12 or 42,

(22) a DNA including a nucleotide sequence encoding an amino acid sequence having 40% or more homology to an amino acid sequence encoded by a nucleotide sequence set forth in SEQ ID NO: 15, 31, or 49,

(23) a DNA including a nucleotide sequence encoding an amino acid sequence having 40% or more homology to an amino acid sequence encoded by a nucleotide sequence set forth in SEQ ID NO: 5, 52, or 51,

(24) a DNA including a nucleotide sequence encoding an amino acid sequence having 40% or more homology to an amino acid sequence encoded by a nucleotide sequence set forth in SEQ ID NO: 20 or 44,

(25) a DNA including a nucleotide sequence encoding an amino acid sequence having 40% or more homology to an amino acid sequence encoded by a nucleotide sequence set forth in SEQ ID NO: 1 or 32,

(26) a DNA including a nucleotide sequence encoding an amino acid sequence having 40% or more homology to an amino acid sequence encoded by a nucleotide sequence set forth in SEQ ID NO: 11 or 29,

(27) a DNA including a nucleotide sequence encoding an amino acid sequence having 40% or more homology to an amino acid sequence encoded by a nucleotide sequence set forth in SEQ ID NO: 10 or 47, and

(28) a DNA including a nucleotide sequence encoding an amino acid sequence having 40% or more homology to an amino acid sequence encoded by a nucleotide sequence set forth in SEQ ID NO: 28 or 422.

2. The composition according to claim 1, wherein the Faecalibacterium prausnitzii is Faecalibacterium prausnitzii having the 28 DNAs (1) to (28).

3. A composition for preventing or treating deterioration in brain function, or for maintaining or improving brain function, including Faecalibacterium prausnitzii, a component of the microorganism, or a culture of the microorganism, having at least one of the following DNAs (1) to (3), as an active ingredient:

(1) a DNA including a nucleotide sequence encoding an amino acid sequence having 40% or more homology to an amino acid sequence encoded by a nucleotide sequence set forth in SEQ ID NO: 22 or 46,

(2) a DNA including a nucleotide sequence encoding an amino acid sequence having 40% or more homology to an amino acid sequence encoded by a nucleotide sequence set forth in SEQ ID NO: 21 or 54,

(3) a DNA including a nucleotide sequence encoding an amino acid sequence having 40% or more homology to an amino acid sequence encoded by a nucleotide sequence set forth in SEQ ID NO: 18 or 55.

4. The composition according to claim 3, wherein the Faecalibacterium prausnitzii is Faecalibacterium prausnitzii having the 3 DNAs (1) to (3).

5. A composition for preventing or treating deterioration in brain function, or for maintaining or improving brain function, including Faecalibacterium prausnitzii, a component of the microorganism, or a culture of the microorganism, having at least one DNA selected from the following DNA group, as an active ingredient:

A group of 150 DNAs each containing a nucleotide sequence according to SEQ ID NOs: 58 to 207 (Note that the nucleotide sequence according to SEQ ID NO: N is a nucleotide sequence encoding an amino acid sequence having 40% or more homology to the amino acid sequence encoded by the nucleotide sequence set forth in SEQ ID NO: N. N is any of 58 to 207.).

6. A composition for preventing or treating deterioration in brain function, or for maintaining or improving brain function, including Faecalibacterium prausnitzii, a component of the microorganism, or a culture of the microorganism, having at least one DNA selected from the following DNA group, as an active ingredient:

A group of 214 DNAs each containing a nucleotide sequence according to SEQ ID NOs: 208 to 421 (Note that the nucleotide sequence according to SEQ ID NO: N is a nucleotide sequence encoding an amino acid sequence having 40% or more homology to the amino acid sequence encoded by the nucleotide sequence set forth in SEQ ID NO: N. N is any of 208 to 421.).

7. The composition according to any one of claims 1 to 6, wherein the brain function is cognitive function.

8. The composition according to any one of claims 1 to 6, which is a food composition.

9. The composition according to any one of claims 1 to 6, which is a pharmaceutical composition.

10. A microorganism which is specified by accession number NITE BP-03169.

11. A microorganism which is specified by accession number NITE BP-03196.

12. A method for evaluating mild cognitive impairment, comprising:

    (1) quantifying a microorganism in subject feces;
    (2) comparing a value obtained by the quantification in step (1) with a corresponding value obtained by quantifying the microorganism in healthy subject feces; and
    (3) determining that the subject is in a state of mild cognitive impairment if, as a result of the comparison in step (2), the quantitative value in the subject feces is lower than the corresponding value, and
    the microorganism is at least one microorganism selected from the group consisting of a microorganism belonging to the genus Faecalibacterium, a microorganism belonging to the genus Anaerostipes, a microorganism belonging to the genus CAG-41, a microorganism belonging to the genus Barnesiella, and a microorganism belonging to the genus Ruminococcus.

13. A method for evaluating mild cognitive impairment, comprising:

    (1) quantifying a microorganism in subject feces;
    (2) comparing a value obtained by the quantification in step (1) with a corresponding value obtained by quantifying the microorganism in healthy subject feces; and
    (3) determining that the subject is in a state of mild cognitive impairment if, as a result of the comparison in step (2), the quantitative value in the subject feces is higher than the corresponding value, wherein the microorganism is at least one microorganism selected from the group consisting of a microorganism belonging to the genus Prevotella.

14. A method for evaluating Alzheimer's disease, comprising:

    (1) quantifying a microorganism in subject feces;
    (2) comparing a value obtained by the quantification in step (1) with a corresponding value obtained by quantifying the microorganism in healthy subject feces; and
    (3) determining that the subject is suffering from Alzheimer's disease if, as a result of the comparison in step (2), the quantitative value in the subject feces is lower than the corresponding value, wherein the microorganism is at least one microorganism selected from the group consisting of a microorganism belonging to the genus Blautia, a microorganism belonging to the genus Fusicatenibacter, a microorganism belonging to the genus Ruminococcus, and a microorganism belonging to the genus Eubacterium.

15. A method for evaluating Alzheimer's disease, comprising:

    (1) quantifying a microorganism in subject feces;
    (2) comparing a value obtained by the quantification in step (1) with a corresponding value obtained by quantifying the microorganism in healthy subject feces; and
    (3) determining that the subject is suffering from Alzheimer's disease if, as a result of the comparison in step (2), the quantitative value in the subject feces is higher than the corresponding value, wherein the microorganism is at least one microorganism selected from the group consisting of a microorganism belonging to the genus Parabacteroides.

Fig. 1

Fig. 2

Fig. 3

Fig. 4

PASSIVE AVOIDANCE TEST (RETENTION TRIAL)

Fig. 5

Fig. 6

PASSIVE AVOIDANCE TEST (RETENTION TRIAL)

Fig. 7

Fig. 8

Fig. 9

Fig. 10

Fig. 11

## INTERNATIONAL SEARCH REPORT

| International application No. |
| --- |
| **PCT/JP2021/044393** |

| A. | CLASSIFICATION OF SUBJECT MATTER |
| --- |
| *A61P 25/28*(2006.01)i; *C12N 1/20*(2006.01)i; *C12Q 1/02*(2006.01)i; *C12Q 1/04*(2006.01)i; *A61K 35/74*(2015.01)i; *A61K 35/741*(2015.01)i; *A23L 33/135*(2016.01)i<br>FI: A23L33/135; C12Q1/02; C12Q1/04; A61P25/28; A61K35/741; A61K35/74 A; A61K35/74 D; A61K35/74 G; C12N1/20 A; C12N1/20 E ZNA |

According to International Patent Classification (IPC) or to both national classification and IPC

| B. | FIELDS SEARCHED |
| --- | --- |

Minimum documentation searched (classification system followed by classification symbols)

A61P25/28; A23L33/135; C12N1/20; C12Q1/02; C12Q1/04; A61K35/74; A61K35/741

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN);GenBank/EMBL/DDBJ/GeneSeq; UniProt/GeneSeq

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | SUN, J. et al. Fecal microbiota transplantation alleviated Alzheimer's disease-like pathogenesis in APP/PS1 trangenic mice. Translational Psychiatry. 2019, vol. 9, 189<br>abstract, p. 4, left column, paragraph [0003] to p. 6, paragraph [0002], fig. 4 | 1-9 |
| X | HAO, Z. et al. Faecalibacterium prausnitzii (ATCC 27766) has preventive and therapeutic effects on chronic unpredictable mild stress-induced depression-like and anxiety-like behavior in rats. Psychoneuroendocrinology. 2019, vol. 104, pp. 132-142<br>abstract | 1-9 |
| X | REN, T. et al. Gut Microbiota Altered in Mild Cognitive Impairment Compared With Normal Cognition in Sporadic Parkinson's Disease. Frontiers in Neurology. 25 February 2020, vol. 11, article 137<br>abstract | 12-13 |

☑ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **21 January 2022** | **08 February 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2021/044393** |

### C. DOCUMENTS CONSIDERED TO BE RELEVANT

| Category* | Citation of document, with indication, where appropriate, of the relevant passages | Relevant to claim No. |
| --- | --- | --- |
| X | CATTANEO, A. et al. Association of brain amyloidosis with pro-inflammatory gut bacterial taxa and peripheral inflammation markers in cognitively impaired elderly. Neurobiology of Aging. 2017, vol. 49, pp. 60-68<br>abstract, p. 63, right column, paragraphs [0002]-[0004], fig. 1 | 14-15 |
| X | HARAN, J. P. et al. Alzheimer's Disease Microbiome Is Associated with Dysregulation of the Anti-Inflammatory P-Glycoprotein pathway. mBio. 2019, vol. 10, e00632-19<br>abstract, p. 3, paragraphs [0002]-[0003], p. 5, paragraph [0003], fig. 2, 3 | 14-15 |
| X | LIU, P. et al. Altered microbiomes distinguish Alzheimer's disease from amnestic mild cognitive impairment and health in a Chinese cohort. Brain, Behavior, and Immunity. 2019, vol. 80, pp. 633-643<br>abstract, p. 636, left column, the last paragraph to right column, the last paragraph, fig. 2 | 14-15 |
| P, X | UEDA, A. et al. Identification of Faecalibacterium prausnitzii stains for gut microbiome-based intervention in Alzheimer's-type dementia. Cell Reports Medicine. 21 September 2021, vol. 2, 100398<br>abstract, etc. | 1-9 |

Form PCT/ISA/210 (second sheet) (January 2015)

## INTERNATIONAL SEARCH REPORT

| International application No. |
| --- |
| **PCT/JP2021/044393** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

<table>
<tr><td>International application No.<br><br>**PCT/JP2021/044393**</td></tr>
</table>

**Box No. III      Observations where unity of invention is lacking (Continuation of item 3 of first sheet)**

This International Searching Authority found multiple inventions in this international application, as follows:

Claims 1-11 are inventions relating to Faecalibacterium prausnitzii used to prevent or treat the deterioration of brain function, or maintain or improve brain function.

Meanwhile, an invention in claim 12 is a method for evaluating mild cognitive impairment by quantifying specific microorganisms in feces, wherein the microorganisms are at least one kind of microorganisms selected from the group consisting of microorganisms belonging to the genus Faecalibacterium, microorganisms belonging to the genus Anaerostipes, microorganisms belonging to the genus CAG-41, microorganisms belonging to the genus Barnesiella, and microorganisms belonging to the genus Ruminococcus, and inventions in claims 1-11 share the common technical feature of microorganisms related to brain function, but said technical feature does not make a contribution over the prior art in light of the disclosure of the following documents 1-6, and thus cannot be said to be a special technical feature.

In addition, claim 12 sets forth a plurality of inventions for evaluating mild cognitive impairment by quantifying microorganisms belonging to the genus Faecalibacterium, microorganisms belonging to the genus Anaerostipes, microorganisms belonging to the genus CAG-41, microorganisms belonging to the genus Barnesiella, or microorganisms belonging to the genus Ruminococcus in feces, but the evaluation of mild cognitive impairment by quantifying microorganisms in feces does not make a contribution over the prior art in light of the disclosure of the following document 3, and thus cannot be said to be a special technical feature. Therefore, it can be said that claim 12 includes five inventions according to what is selected as a microorganism to be quantified.

This is also applied to an invention in claim 13.

In addition, an invention in claim 14 is a method for evaluating Alzheimer's disease by quantifying specific microorganisms in feces, wherein the microorganisms are at least one kind of microorganisms selected from the group consisting of microorganisms belonging to the genus Blautia, microorganisms belonging to the genus Fusicatenibacter, microorganisms belonging to the genus Ruminococcus, and microorganisms belonging to the genus Eubacterium, and inventions in claims 1-11 share the common technical feature of microorganisms related to brain function, but said technical feature does not make a contribution over the prior art as described above, and thus cannot be said to be a special technical feature.

In addition, claim 14 sets forth a plurality of inventions for evaluating Alzheimer's disease by quantifying microorganisms belonging to the genus Blautia, microorganisms belonging to the genus Fusicatenibacter, microorganisms belonging to the genus Ruminococcus, or microorganisms belonging to the genus Eubacterium in feces, but the evaluation of Alzheimer's disease by quantifying microorganisms in feces does not make a contribution over the prior art in light of the disclosure of the following documents 4-6, and thus cannot be said to be a special technical feature. Therefore, it can be said that claim 14 includes four inventions according to what is selected as a microorganism to be quantified.

This is also applied to an invention in claim 15.

Accordingly, it can be said that this application includes 12 inventions.

## INTERNATIONAL SEARCH REPORT

| International application No. |
| --- |
| **PCT/JP2021/044393** |

**Box No. III      Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

47

INTERNATIONAL SEARCH REPORT
Information on patent family members

International application No.

PCT/JP2021/044393

| Patent document cited in search report | Publication date (day/month/year) | Patent family member(s) | Publication date (day/month/year) |
|---|---|---|---|

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MINTER MR et al.** *Sci Rep,* 21 July 2016
- **HARACH T et al.** *Sci Rep,* 08 February 2017, vol. 7, 41802
- **SAJI N. et al.** *Sci Rep,* 30 January 2019, vol. 9 (1), 1008
- **HAO Z. et al.** *Psychoneuroendocrinology,* June 2019, vol. 104, 132-142
- *Bioinformatics,* 15 November 2015, vol. 31 (22), 3691-3693
- *Genome Biol,* 06 August 2015, vol. 16 (1), 157